# EUROPEAN PATENT SPECIFICATION

(11) **EP 2 646 554 B1**
(45) Date of publication and mention of the grant of the patent: **08.06.2016**
(21) Application number: 11845889.2
(22) Date of filing: 30.11.2011
(51) Int. Cl.: C12N 15/11, C12Q 1/68, G01N 33/50

(54) **PREDICTION OF SPONTANEOUS PRETERM BIRTH BY MEASURING CELL FREE NUCLEIC ACIDS IN MATERNAL BLOOD**
VORHERSAGE SPONTANER FRÜHGEBURTEN DURCH MESSUNG ZELLFREIER NUKLEINSÄUREN IM MÜTTERLICHEN BLUT
PRÉDICTION D'UNE NAISSANCE PRÉMATURÉE SPONTANÉE EN MESURANT LA TENEUR EN ACIDES NUCLÉIQUES ACELLULAIRES DANS LE SANG MATERNEL

(30) Priority: 30.11.2010 US 418375 P; 30.11.2010 US 418368 P
(43) Date of publication of application: 09.10.2013
(73) Proprietor: Dong, Yafeng, Overland Park KS 66221 (US); Weiner, Carl, Mission Hills KS 66208 (US)
(72) Inventor: Dong, Yafeng, Overland Park KS 66221 (US); Weiner, Carl, Mission Hills KS 66208 (US)
(74) Representative: Kaminski Harmann
(86) International application number: PCT/US2011/062661
(87) International publication number: WO 2012/075150

(56) References cited:
- WO-A2-2009/093254
- US-A1- 2008 090 759
- US-A1- 2008 254 454
- US-A1- 2010 112 581
- US-B2- 7 807 366
- MAYOR-LYNN K ET AL: "Expression profile of microRNAs and mRNAs in human placentas from pregnancies complicated by preeclampsia and preterm labor", REPRODUCTIVE SCIENCES 2011 SAGE PUBLICATIONS INC. USA, vol. 18, no. 1, 15 November 2010 (2010-11-15), pages 46-56, XP002729181, ISSN: 1933-7191
- YOGEV Y ET AL: "110: Spontaneous preterm labor- a possible role for micro-RNA", AMERICAN JOURNAL OF OBSTETRICS & GYNECOLOGY, MOSBY, ST LOUIS, MO, US, vol. 197, no. 6, 1 December 2007 (2007-12-01), page S44, XP022591296, ISSN: 0002-9378, DOI: 10.1016/J.AJOG.2007.10.121 [retrieved on 2007-12-01]
- YOGEV Y ET AL: "459: Micro RNA: A central new player in post-transcriptional regulation pathway in preeclampsia", AMERICAN JOURNAL OF OBSTETRICS & GYNECOLOGY, MOSBY, ST LOUIS, MO, US, vol. 197, no. 6, 1 December 2007 (2007-12-01), page S135, XP022591645, ISSN: 0002-9378, DOI: 10.1016/J.AJOG.2007.10.478 [retrieved on 2007-12-01]
- CHIM STEPHEN S C ET AL: "Detection and characterization of placental microRNAs in maternal plasma", CLINICAL CHEMISTRY, AMERICAN ASSOCIATION FOR CLINICAL CHEMISTRY, WASHINGTON, DC, vol. 54, no. 3, 1 March 2008 (2008-03-01), pages 482-490, XP002518104, ISSN: 0009-9147, DOI: 10.1373/CLINCHEM.2007.097972 [retrieved on 2008-01-24]
- FARINA ET AL: "High levels of fetal cell-free DNA in maternal serum: A risk factor for spontaneous preterm delivery", AMERICAN JOURNAL OF OBSTETRICS & GYNECOLOGY, MOSBY, ST LOUIS, MO, US, vol. 193, no. 2, 1 August 2005 (2005-08-01), pages 421-425, XP005079561, ISSN: 0002-9378, DOI: 10.1016/J.AJOG.2004.12.023
- LITTON C ET AL: "Noninvasive prenatal diagnosis: Past, present, and future", MOUNT SINAI JOURNAL OF MEDICINE 2009 JOHN WILEY AND SONS INC. USA, vol. 76, no. 6, December 2009 (2009-12), pages 521-528, XP002729182, ISSN: 0027-2507
- WRIGHT C F ET AL: "The use of cell-free fetal nucleic acids in maternal blood for non-invasive prenatal diagnosis", HUMAN REPRODUCTION UPDATE, OXFORD UNIVERSITY PRESS, OXFORD, GB, vol. 15, no. 1, 1 January 2009 (2009-01-01), pages 139-151, XP002613058, ISSN: 1355-4786, DOI: 10.1093/HUMUPD/DMN047 [retrieved on 2008-10-22]

## Description

### BACKGROUND

Preterm birth remains a major societal problem due to the short and long term health complications of the preterm infants. Many preterm infants live the initial parts of their lives in intensive and critical care units, and often have excess health problems through adulthood compared to infants delivered at term. Approximately 12% of infants delivered are a product of a preterm birth (PTB), which can be characterized as a spontaneous birth before 37 weeks of pregnancy. PTB is also associated with >70% of neonatal deaths and nearly half of long-term neurologic disabilities. Despite great effort among all health sectors, the PTB rate has continued to increases. Accordingly, there remains a great need to identify women at risk of having a PTB and to better understand the mechanisms culminating in PTB.

Mayor-Lynn K. et al: "Expression profile of microRNAs and mRNAs in human placentas from pregnancies complicated by preeclampsia and preterm labor", Reproductive Sciences 2011 Sage Publications Inc. USA, vol. 18, no. 1, 15 November 2010, pages 46-56 disclose studies of the expression profile of microRNAs and mRNAs in placentas of women with spontaneous preterm birth, preeclampsia and healthy control at term, whereby it was found that the placental transcriptome differs among the 3 aforementioned groups.

WO 2009/093254 A2 contains teachings related to serum microRNA data from women with preeclampsia, and describes several microRNAs that differ in their expression levels from those in healthy women, wherein the RNA is always obtained from maternal serum samples.

Yogev Y. et al: "110: Spontaneous preterm labor - a possible role for micro-RNA", American Journal of Obstetrics & Gynecology, Mosby, St. Louis, MO, US, vol. 197, no. 6, 1 December 2007, page S44 and Yogev Y. et al: "459: MicroRNA: A central new player in post-transcriptional regulation pathway in preeclampsia", American Journal of Obstetrics & Gynecology, Mosby, St. Louis, MO, US, vol. 197, no. 6, 1 December 2007, page S135 disclose a study of women in spontaneous preterm labor and in term labor, each delivered by cesarean section. MicroRNA was extracted from myometrium, placenta, amniotic fluid and maternal serum and then quantified, whereby differences in microRNA expression between women in spontaneous preterm and term labor have been found.

Chim Stephen S. C. et al: "Detection and characterization of placental microRNAs in maternal plasma", Clinical Chemistry, American Association for Clinical Chemistry, Washington, DC, vol. 54, no. 3, 1 March 2008, pages 482-490 disclose the detection and characterization of particular placental microRNAs in maternal plasma and conclude that these pregnancy associated microRNAs can be suitable for pregnancy monitoring.

Wright C. F. et al: "The use of cell-free fetal nucleic acids in maternal blood for non-invasive prenatal diagnosis", Human Reproduction update, Oxford University Press, Oxford, GB, vol. 15, no. 1, 1 January 2009, pages 139-151 disclose the use of particular cell-free nucleic acids in maternal blood, originating from the fetus and detected during pregnancy, for non-invasive prenatal diagnosis.

### BRIEF DESCRIPTION OF THE FIGURES

The foregoing and following information as well as other features of this disclosure will become more fully apparent from the following description and appended claims, taken in conjunction with the accompanying drawings. Understanding that these drawings depict only several embodiments in accordance with the disclosure and are, therefore, not to be considered limiting of its scope, the disclosure will be described with additional specificity and detail through use of the accompanying drawings, in which:
Figures 1A-1D illustrate that our new discovered messenger RNA (mRNA) normalization sequences of PPIA are more stabilized (Figures 1D) compared to published normalization sequences (Figures 1A, 1B, and 1C);
Figures 2A-2D illustrate that our new discovered snRNA:U6 is not impacted by different gestational age; snRNA:U6 plays a better role as micro RNA (miRNA) normalization sequences compared to reported sequences;
Figure 3 illustrates results of a high through-put gene Real-time PCR platform validated microarray selected CFP miRNA as PTB biomarkers;
Figures 4A-4B illustrates that CFP miRNA PTB biomarkers can be altered by gestation, MIR-99a can be triggered as early as 16 weeks;
Figure 5A includes a plasmid DNA reconstruction containing one of the CFP mRNA-APOA4;
Figure 5B includes an image of a gel electrophoresis illustrates that the APOA4 Vector reconstruction is successful;
Figure 5C illustrates that myometrium cell APOA4 protein can be up-regulated by APOA4 plasmid DNA; and
Figure 5D illustrates that CFP mRNA biomarker-APOA4 can trigger intracellular Ca²⁺ concentration in myometrium cell consistent with enhanced contractility.

### DETAILED DESCRIPTION

In the following detailed description, reference is made to the accompanying drawings, which form a part hereof. In the drawings, similar symbols typically identify similar components, unless context dictates otherwise. The illustrative embodiments described in the detailed description, drawings, and claims are not meant to be limiting. Other embodiments may be utilized, and other changes may be made, without departing from the spirit or scope of the subject matter presented herein. It will be readily understood that the aspects of the present disclosure, as generally described herein, and illustrated in the figures, can be arranged, substituted, combined, separated, and designed in a wide variety of different configurations, all of which are explicitly contemplated herein.

Generally, the present invention relates to the use of nucleic acids to predict preterm birth (PTB) or determine the probability or susceptibility of PTB in a woman. The nucleic acids useful for PTB diagnostics can include nucleic acid primers and/or probes that bind with specific nucleic acid sequences as well as the nucleic acids that are increased or decreased in a woman that may be susceptible to PTB. The nucleic acids can include specific nucleic acid sequences relevant to PTB, which sequences function as biomarkers for PTB. Diagnostic kits can be provided with specific nucleic acid primers and/or probes, labeled or unlabeled, that can selectively bind with nucleic acids associated with PTB. The diagnostic kits can also include nucleic acid primers that can be used for amplifying nucleic acids associated with PTB. The diagnostic kits can include probes that can identify the presence of certain nucleic acid sequences. In one aspect, all PTB specific nucleic acids can be included on customized PCR cards. By utilizing high throughput PCR technique, the PCR cards can be used for diagnostics and determination of nucleic acid presence and/or amount. The methods of the present invention can include diagnosing whether or not a pregnant woman is susceptible to PTB.

The present invention can also include normalization nucleic acids (e.g., mRNA and miRNA) that have normalization sequences that can be used to normalize the relative levels of nucleic acid data from one sample to the next. We illustrate that our new discovered mRNA and miRNA normalization sequence are stabilized and not impacted by gestational age compared to previously published reports. These normalization nucleic acids can be also be included in diagnostic kits. The methods of the present invention can use the normalization nucleic acids in sample normalization protocols. These protocols can be useful for normalizing nucleic acid amounts between samples. While these normalization nucleic acids are useful for PTB diagnostic protocols, they can also be used to normalize nucleic acid amounts for any purpose.

While RNA is a preferred nucleic acid for the compositions and methods described herein, it is possible that DNA or RNA/DNA hybrids could also be used as nucleic acid probes and/or primers for diagnostics or normalization protocols. However, the nucleic acids that are identified to be present, up-regulated, or down-regulated in diagnostic protocols will generally be RNA as it is transcribed from DNA (e.g., complementary RNA) or as processed into mRNA. The RNA may also be regulatory RNA, such as non-coding small RNA (miRNA, siRNA, snRNA, or snoRNA) that are involved in gene silencing or transcription or translation regulation. Also, normalization protocols will generally be performed with RNA.

The nucleic acids can be cell free plasma (CFP) RNA, which refers to RNA derived from a variety of cells within differing organs, and circulates systemically. CFP RNA may include several types including coding RNA (e.g., mRNA) and non-coding RNAs (e.g., siRNA, miRNA, snoRNA, snRNA). Using microarray techniques, we screen all gene mRNA and non-coding RNAs including siRNA, miRNA, snoRNA, and snRNA. We found only some mRNA and miRNA can be altered by preterm labor. In one aspect, the CFP RNAs can include maternal CFP mRNA and CFP miRNA. The CFP RNAs can be detectable in plasma from the mother's peripheral circulation long before any symptoms or signs of preterm labor. The nucleic acids can be characterized as CFP RNA PTB biomarkers as they can individually or in combination provide a biomarker for PTB and prediction of PTB or PTB susceptibility. The CFP RNA PTB biomarkers can be used to provide a pattern of PTB biomarkers that may reflect the underlying mechanisms that result in PTB or susceptibility thereto.

In one embodiment, the CFP RNA can be specific RNA nucleic acid sequences. That is, the sequences can be a whole or portion of an mRNA or miRNA. The sequences themselves can be used for preparing primers and/or probes for the methods described herein, and may be used at targets for detection as well as for further studies in developing targeted therapies. The CFP RNA nucleic acid sequences are provided in the Sequence Listing and have SEQ ID NOs: 1-303. These sequences in the Sequence Listing are provided in DNA format; however, these sequences can be employed with the RNA format with uracil (U) replacing thymine (T). Accordingly, references to the SEQ ID NOs 1-303 of the Sequence Listing can be in RNA format, DNA format, or DNA/RNA hybrid. In a preferred embodiment, the SEQ ID NOs 1-303 of the Sequence Listing are specifically RNA, such as for the miRNA and mRNA described herein, and thereby any "T" can be replaced with a "U" as understood by one of ordinary skill in the art. Thus, a recitation of SEQ ID NOs 1-303 of the Sequence Listing can specifically refer to the corresponding RNA nucleic acids.

Accordingly, CFP RNA PTB biomarkers can be used for the development of targeted pharmacotherapy that could be initiated before myometrial activation occurs, as opposed to after the onset of symptoms such as cervical shortening or contractions. The CFP RNA PTB biomarkers can be used in order to design a therapy that can modulate the production of certain biological substances, such as proteins associated with myometrial activation or the inhibition of myometrial activation. The PTB biomarkers may also be used in diagnostic protocols for other pregnancy disorders, such as abnormal placentation (e.g., preeclampsia, IUGR, etc.), dysfunctional cervical ripening, short cervix, or others where the pathologic mechanisms overlap or intersect. The PTB biomarkers can be used to identify maternal CFP transcriptome patterns indicative of certain fetal malformations, such as for diagnosis of common triploidies.

In one embodiment, the present invention can use a combination of CFP RNA PTB biomarkers for diagnosing a pregnancy disorder or susceptibility thereof, and providing a therapy in order to treat and/or prevent the pregnancy disorder. For example, a diagnostic protocol can be used to diagnose or predict the ultimate development of a sonographically short cervix, and then a medical professional can treat the condition with progesterone supplementation from information obtained from the PTB biomarkers, which diagnosis and treatment could be as early as 12, 16, 18, or 22 weeks gestation before the cervix has actually shortened.

In one embodiment, a diagnostic kit can be provided with one or more CFP RNA PTB biomarkers and instructions of use that can be used to identify susceptibility of PTB in women as early as possible (e.g., 12, 16, 18, 20, 22, 24, 26, 28, 30, or up to 32 weeks) to allow for intervention before a PTB indicator such as either myometrial activation or cervical ripening or both is irrevocably activated. The diagnostic kit can include one or multiple PTB biomarkers in a single composition or PCR card or PCR card spot, where each PTB biomarker can be used for targeting different causes of PTB. Alternatively, two or more of such PTB biomarkers may be used together to maximize the predictive values of the test. The diagnostic kit can include nucleic acids that are the complement of CFP RNA PTB biomarker sequences that are used to perform the diagnostic. These nucleic acids can be the primers and/or probes for such a diagnostic protocol. The nucleic acids can also be included in plasmids for expression of the PTB biomarker sequences. The diagnostic kit can also identify the CFP RNA PTB biomarker sequences that are to be identified as up-regulated or down-regulated, and may specify the mRNA, miRNA, general sequence thereof, or the exact sequences in such CFP RNA PTB biomarkers that are specific to which the primers and/or probes hybridize. The CFP RNA PTB biomarkers have sequences that are included in the Sequence Listing having SEQ ID NOs: 5-300. In some instances, such as shorter sequences, the entire recited sequence can be used, and in other instances unique portions of the sequences that are unique and specific for that mRNA or miRNA can be used in the invention described herein.

### NORMALIZATION SEQUENCES

Quantification of nucleic acids (e.g., RNA) extracted from a biological sample can be important data. The actual quantification of RNA in a sample and its comparison to other RNA sequences in a single sample or in multiple samples usually requires a nucleic acid normalization sequence. The normalization sequence can be RNA that has an amount or expression level is generally stable under the conditions studied. That is, the normalization sequence can have an amount or level that is substantially unaffected by any physiological circumstances present in a subject, and thereby the normalization sequence can be used to normalize the amount of nucleic acid in separate samples for comparison. The separate samples can be from different subjects or the same subject at different time points, such as different time points in pregnancy. For example, the normalization sequence can be used to normalize the amount of RNA in Q-rtPCR studies, such as by normalizing the amount of the RNA sequence of interest. The normalization sequences described herein can be used alone or in combination, and may be used to normalize samples to be assayed for PTB biomarkers. However, the normalization sequences can be used to normalize the amount of RNA in different samples for other purposes than for PTB biomarkers. Thereby, the normalization sequences can be used as general normalization sequences to normalize the amount of RNA in different samples for any purpose. Thus, the normalization sequences provided herein can be for quantification of free RNA isolated from biological samples.

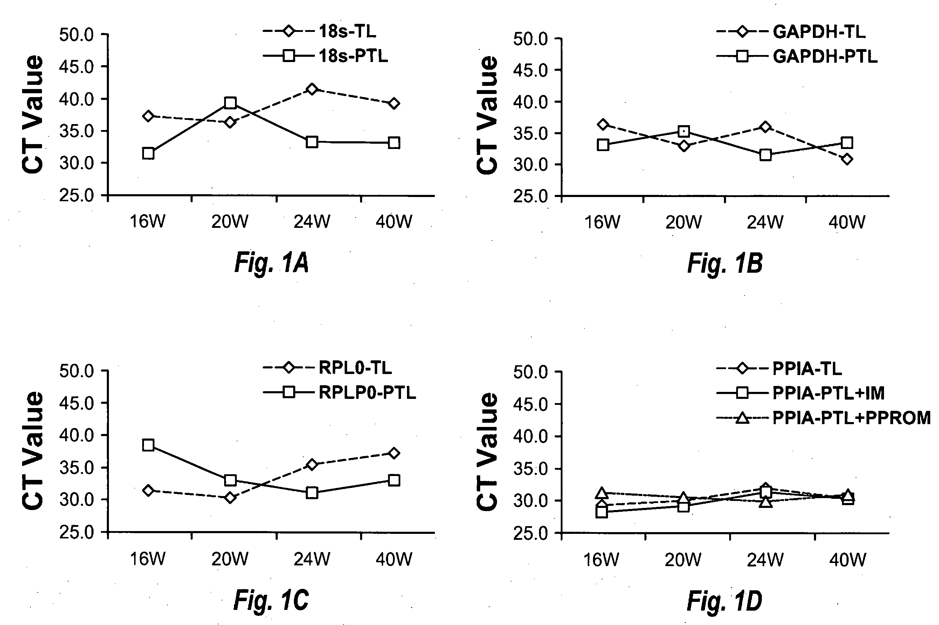

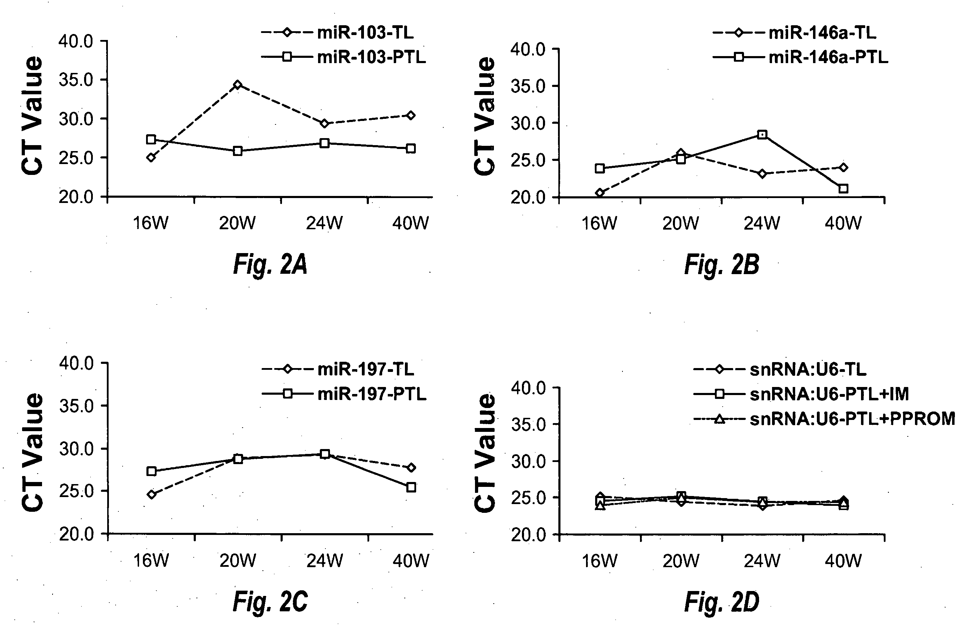

It has been determined that previously reported normalization sequences utilized in other tissues for quantification of isolated RNA (e.g., mRNA: 18s RNA, RPLP0, GAPDH; miRNA: miR-103, miR-146a, and miR-197) were either expressed inconsistently in control plasma samples or were altered by either pregnancy, gestational age or disease (see Figures 1A-1C and 2A-2C). Thus, new normalization sequences were sought and identified (see Figures 1D, and 2D). These new normalization sequences can include CFP mRNA and CFP miRNA sequences that are substantially unchanged by any condition, such as by pregnancy. However, the CFP RNA normalization sequences and related process can be equally applicable to almost any disease state ranging from pregnancy and PTB to malignancy to cardiovascular disease to bone disease or joint disease or the like.

In one embodiment, the normalization sequence includes a circulating RNA. Such a normalization sequence can be described as human (i.e., *homo sapiens*) peptidylprolyl isomerase A (i.e., cyclophilin A, rotmase A), which is encoded by the PPIA gene. The normalization sequence can be the mRNA for peptidylprolyl isomerase. The peptidylprolyl isomerase normalization sequence can be found at accession number: NM_021130 and/or NM_001008741. The peptidylprolyl isomerase normalization sequence is defined herein as SEQ ID NO: 1), and can be useful for normalization of mRNA.

In one embodiment, the normalization sequence can include miRNA. Such a normalization sequence can be a *Drosophila melanogaster* small nuclear RNA, such as snRNA:U6. The snRNA:U6 normalization sequence can be snRNA:U6 at 96Aa, 96:Ab, and/or 96Ac. These normalization sequences can be described as snRNA:U6:96Aa (SEQ ID NO: 2 for miRNA), snRNA:U6:96Ab (SEQ ID NO: 3 for miRNA), and/or snRNA:U6:96Ac (SEQ ID NO: 4 for miRNA), and can be found at the following accession numbers, respectively: NR_002081 (snRNA:U6:96Aa); NR_002082 (snRNA:U6:96Ab); and NR_002083 (snRNA:U6:96Ac). Figures 1A-1D and 2A-2D illustrate the impact of gestational age, preterm premature rupture of membranes (PPROM) and ultimate spontaneous preterm birth on some of the sequences rejected and the one mRNA and miRNA selected for normalization (see Figures 1D and 2D). Accordingly, SEQ ID NOs: 2-4 for miRNA, and SEQ ID No 1 for mRNA can be used for normalization sequences generally, and particularly for normalization of PTB biomarkers. Primers and probes for these sequences can be readily obtained by one of ordinary skill in the art with this application. For example, sequences for the forward primer, reverse primer, and probe for SEQ ID NO: 1 (e.g., for mRNA normalization sequence of PPIA) will be: Forward primer: GCTTTGGGTCCAGGAATGG - SEQ ID NO: 301; Reverse primer: GTTGTCCACAGTCAGCAATGGT - SEQ ID NO: 302; and Probe: AGACCAGCAAGAAGAT - - SEQ ID NO: 303, which can also be considered normalization sequences for the invention recited herein.

In one embodiment, a normalization kit can be provided that includes one or more of these normalization sequences in nucleic acid format, such as RNA, DNA, or RNA/DNA hybrid. Preferably, the sequences of the normalization kit will include the complement of the sequences recited in the SEQ ID NO: 1-4. Also preferably, the sequences of the normalization kit will include the sequences recited in SEQ ID NO: 301-303 as these sequences are complementary to SEQ ID NO 1. Also, the normalization kit may also be included in a PTB diagnostic kit as described herein. The normalization kit can include individual compositions that have a single normalization sequence, or a single composition can include one, two, three, or all four of the normalization sequences and/or primers and/or probes thereof. Each sequence may be on a separate nucleic acid, or multiple sequences can be on a single nucleic acid. The normalization sequences can be provided with or without a label, such as a visual label or radiolabel. The normalization sequences can be provided on a customized PCR card or similar device configured for use in nucleic acid detection and/or quantification and/or qualification, which card or similar device can be configured as a high-throughput Real-time Q-PCR system. One or more sample spots on a customized PCR card can have one, two, three, or all four of the normalization sequences and/or the primers and/or probes thereof. For example, the PCR card can have one spot with one normalization sequence or a spot with up to all four normalization sequences and/or primers and/or probes thereof. Such a PCR card can have one or more normalization sequences spots, which spots can be reaction wells or the like. The PCR card may also have assay spots having nucleic acids to be assayed. For example, the customized PCR card can be configured as an ABI high-through put Real-time PCR system. The incorporation of these normalization sequences in the various PCR card products allows them to be more readily used for plasma-derived samples, and in repeated measures of CFP mRNA and CFP miRNA or other nucleic acid normalization.

In one embodiment, a normalization sequence can be a nucleic acid that contains or consists of the sequence. The normalization sequence can be identical to one of SEQ ID NOs: 2-4 for miRNA, and SEQ ID NO 1 for mRNA as well as SEQ ID NOs: 301-303, or can be a complement thereof, sense or antisense, as well as a sequence that hybridizes therewith under suitable conditions. The normalization sequence can have perfect complementarity or greater than or about 95% complementarity, greater than or about 90% complementarity, greater than or about 85% complementarity, or greater than or about 80% complementarity. Complementarity can be considered with respect to a nucleic acid in a biological sample or natural nucleic acid obtained therefrom. The normalization sequence can be a continuous or it can have one or more bulges or mismatches upon hybridization. The normalization sequence can also include one or more chemical modifications, such as a 2' carbon modification. The normalization sequence may or may not form an overhang upon hybridization. The normalization sequence can include a sequence from about 15 nucleotides to the full sequence, from about 16 nucleotides to about 100 nucleotides, from about 17 nucleotides to about 50 nucleotides, from about 18 nucleotides to about 30 nucleotides, from about 19 nucleotides to about 25 nucleotides, or from about 20 to about 22 nucleotides in sequence of one of SEQ ID NOs: 2-3 for miRNA, and SEQ ID 1 for mRNA. The normalization sequence can include a unique sequence segment or complement thereof of the full sequence having a length as described.

In one embodiment, the present invention can include a method of identifying a normalization sequence, such as a pregnancy normalization sequence. The method can include obtaining a plurality of plasma free (e.g., CFP) RNA, CFP mRNA, and/or CFP miRNA sequences from a plurality of subjects (e.g., men or women) prior to a particular disease state (e.g. spontaneous preterm birth in women or prostate cancer in men, without limitation thereto). When pregnant women, the sequences can be obtained prior to or at 32 weeks, 30 weeks, 28 weeks, 26 weeks, 24 weeks, 22 weeks, 20 weeks, 18 weeks, 16, or 12 weeks of pregnancy, and possibly even earlier in pregnancy. Once obtained, one or more CFP mRNA and/or CFP miRNA sequences that are unchanged between disease states (e.g. between two or more women destined for a spontaneous preterm birth of less than 32 weeks) can be identified, and these unchanged sequences can be determined to be normalization sequences. Different disease states can be prior to onset of a disease and then after onset of disease. The identified sequences can be assayed and confirmed to be CFP mRNA and/or CFP miRNA or other CFP RNA that are substantially unchanged between two or more of the samples. The unchanged sequences can be further confirmed to be unchanged between additional sequences. The unchanged sequences can be normalization sequences as described herein.

Another embodiment of a method of identifying a CFP normalization nucleic acid can include obtaining a plurality of plasma free (e.g., CFP) mRNA or miRNA sequences from a plurality of nonpregnant women or pregnant women prior to 32 weeks, such as between about 12-32 weeks of pregnancy. The sequences can be from one woman that is or becomes pregnant or from a plurality of women that are or become pregnant, where one or more sequences can be from a woman that becomes pregnant and that is susceptible to PTB. One or more sequences can even be after birth or after a PTB. After obtained, the sequences can be assayed in order to identify one or more plasma cell free mRNA or miRNA sequences unchanged between different pregnancy states. The different pregnancy states can be between two or more women, or between nonpregnant and pregnant, or between early pregnancy (e.g., before about 16 weeks), or late pregnancy (e.g., after about 16 weeks), or between prior to onset of a PTB-indicating symptom or after a PTB-indicating symptom, or between pregnancy and having or had preterm birth of less than 32 weeks, or combination thereof. The sequences can then be analyzed in order to confirm (e.g., by Qrt-PCR) that the CFP mRNA or miRNA are unchanged between two or more samples having the sequences. The analysis can be between different women or different pregnancy states. Unchanged sequence presence or amount of sequence is indicative that the sequence can be a normalization sequence as described herein.

In another embodiment, a method of identifying CFP normalization nucleic acids or sequences thereof can include: obtaining a plurality of CFP mRNA or CFP miRNA sequences from a plurality of women between 16-28 weeks of pregnancy or prior to birth or PTB; identifying one or more CFP mRNA or miRNA sequences unchanged between two or more women having, had, or that will have PTB of less than 32 weeks; and confirming, by Qrt-PCR, that the CFP mRNA or miRNA is unchanged between two or more samples of CFP RNA and/or CFP miRNA from one or more other women that are un-pregnant, pregnant or two or more women having, had, or that will have PTB of less than 32 weeks. Also, a plurality of CFP RNA can be obtained from women after having a term birth or a PTB.

In one embodiment, the unchanged sequences or possible normalization sequences can be assayed by confirming the sequences to be unchanged or normalization sequences between randomly selected samples.

In one embodiment, the present invention includes a method of quantification of CFP RNA. Such a method can include providing a CFP normalization nucleic acid, and comparing a sample of purified plasma RNA (CFP RNA) from a subject with the CFP normalization sequence, such as a nucleic acid having the normalization sequence. Such a comparison can then be used to determine the amount of CFP RNA in the sample and across two or more samples. Accordingly, different samples from different sources can be normalized using the CFP normalization sequence. One, two, three, or four of the different normalization sequences and/or primers and/or probes thereof can be used for quantification of CFP RNA. The method of quantification of CFP RNA can be performed substantially as known or later developed by using the normalizations sequences described herein.

In another embodiment, a method of normalizing CFP normalization nucleic acids or sequences thereof can include: obtaining a plurality of CFP mRNA or CFP miRNA sequences from a plurality of women between 12 and 32 weeks or 16-28 weeks of pregnancy or prior to birth or PTB; providing one or more CFP mRNA or miRNA sequences unchanged between two or more women having, had, or that will have PTB of less than 32 weeks; and normalizing the CFP mRNA or miRNA sequences with the known unchanged CFP mRNA or miRNA sequences.

In one embodiment, a method of normalizing CFP mRNA or miRNA sequences can include normalizing with one or more of SEQ ID NOs: 1-4 or primer and/or probe thereof or SEQ ID NOs: 301-303 via standard normalization protocols.

The methods described can also include obtaining samples that have RNA from a subject and processing the sample in order to obtain CFP RNA.

### PTB BIOMARKER SEQUENCES

Quantification of PTB biomarker nucleic acids (e.g., RNA) extracted from a biological sample can be used in order to determine whether or not a pregnant woman is susceptible to PTB. Accordingly, identification of PTB biomarkers can be important in order to diagnose PTB susceptibility or predict PTB. The present invention generally includes new RNA biomarkers and processes to identify plasma RNA biomarkers, and use of the RNA biomarkers to identify pre-disease states related to PTB. The present invention can use RNA biomarkers associated with pregnancy disease states in order to predict whether a pregnant women may develop or become susceptible to developing a particular disease state that may cause PTB. Generally, the PTB biomarkers include nucleic acids that are CFP RNA as described herein.

CFP RNA biomarkers can include maternal and fetal derived RNA sequences. Since myometrial activation can result in spontaneous birth, and since myometrial quiescence is a genomically rich period, changes in the CFP transcriptome (e.g., RNA transcriptome) can be used to predict spontaneous PTB. Such a change in the CFP transcriptome can be indicative of PTB regardless of whether the stimulus originated in either the maternal or fetal compartments. The CFP RNA PTB biomarkers have now been identified and are provided in the Sequence Listing as SEQ ID NOs: 5-300. These CFP RNA PTB biomarker sequences are involved in the biological and regulatory process of pregnancy, and modulation of these CFP RNA PTB biomarkers can be an indication of disease. Also, modulation of these CFP RNA PTB biomarker may be used to inhibit, prevent, or treat a disease associated with the particular mRNA or miRNA of the CFP RNA PTB biomarker.

Briefly, CFP mRNA was obtained at 26-28 weeks from 5 randomly selected women destined for PTB (e.g., birth <32 weeks) absent PPROM (i.e., preterm, premature rupture of membranes), and from 5 control women destined for delivery at term. In a 'Discovery Phase' of CFP mRNA identification, the extracted RNA were run on the Affymetrix Human Whole-Transcript Expression Array, and the mRNA sequences altered in women destined for PTB were identified based on fold change (e.g., ≥1.5x, a standard cutoff used across science) and p value from control (p<0.01). The CFP mRNA were ordered by narrowness of distribution (e.g., Ingenuity Systems Pathway Analysis) since a narrow distribution is a highly desirable test characteristic for any selected marker, where the narrower the distribution of disease and normal, the smaller the overlap in population distributions. Of the 25,934 RNA sequences identified to comprise the CFP transcriptome at 26 weeks, 88 CFP mRNA PTB biomarkers were altered in women destined for PTB; 22 CFP mRNA PTB biomarkers (SEQ ID NOs: 19-41) were up-regulated and 66 CFP mRNA (SEQ ID NOs: 42-106) were down-regulated. Genomic mapping revealed the CFP mRNA PTB marker sequences were associated with expression, cell growth and proliferation, cell cycle, cell death, and cellular assembly and organization.

CFP RNA PTB biomarkers can include but are not limited to non-coding RNA, such as miRNA and snRNA and snoRNA, and others are mRNA. In one embodiment, the CFP RNA PTB biomarkers can include a biomarker that indicates susceptibility to PTB. These CFP RNA PTB biomarkers can include: (SEQ ID NO: 19) *Homo sapiens* taspase, threonine aspartase, 1 (TASP1), mRNA, accession number NM_017714; (SEQ ID NO: 20) *Homo sapiens* zinc finger protein 99 (ZNF99), mRNA, accession numbers NM_001080409 and XM_001132267; (SEQ ID NO: 21) *Homo sapiens* cDNA FLJ16171 fis, clone BRHIP2003272, accession number AK131247; (SEQ ID NO: 22) *Homo sapiens* regenerating islet-derived 3 gamma (REG3G), transcript variant 1, mRNA, accession number NM_001008387; (SEQ ID NO: 23) *Homo sapiens* olfactory receptor, family 51, subfamily A, member 2 (OR51A2), mRNA, accession numbers NM_001004748 and XM_377159; (SEQ ID NO: 24) *Homo sapiens* NADH dehydrogenase (ubiquinone) 1 alpha subcomplex, 2, 8kDa (NDUFA2), nuclear gene encoding mitochondrial protein, transcript variant 1, mRNA, accession number NM_002488; (SEQ ID NO: 25) *Homo sapiens* splicing factor 3a, subunit 3, 60kDa (SF3A3), mRNA, accession number NM_006802; (SEQ ID NO: 26) *Homo sapiens* late cornified envelope 2A (LCE2A), mRNA, accession number NM_178428; (SEQ ID NO: *27) Homo sapiens* S100 calcium binding protein A14 (S100A14), mRNA, accession number NM_020672; (SEQ ID NO: 28) *Homo sapiens* six transmembrane epithelial antigen of the prostate 1 (STEAP1), mRNA, accession numbers NM_012449 and XM_940149; (SEQ ID NO: 29) *Homo sapiens* cDNA FLJ11733 fis, clone HEMBA1005426, accession number AK021795; (SEQ ID NO: 30) *Homo sapiens* speedy homolog E1 (Xenopus laevis) (SPDYE1), mRNA, accession numbers NM_175064, XM_938448, XM_943679, XM_943682, XM_943684, XM_943688, and XM_943692; (SEQ ID NO: 31) *Homo sapiens* tripartite motif-containing 48 (TRIM48), mRNA, accession number NM_024114; (SEQ ID NO: 32) *Homo sapiens* non-protein coding RNA 152 (NCRNA00152), transcript variant 1, non-coding RNA, accession numbers NR_024204, XR_042051, and XR_042052; (SEQ ID NO: 33) *Homo sapiens* cDNA FLJ39739 fis, clone SMINT2016440, accession number AK097058; (SEQ ID NO: 34) *Homo sapiens* FXYD domain containing ion transport regulator 2 (FXYD2), transcript variant c, mRNA, accession number NM_001127489; (SEQ ID NO: 35) *Homo sapiens* chromosome 1 open reading frame 104, mRNA (cDNA clone MGC:70363 IMAGE:5183308), complete cds, accession number BC062571; (SEQ ID NO: 36) *Homo sapiens* phosphoserine aminotransferase 1 (PSAT1), transcript variant 1, mRNA, accession number NM_058179; (SEQ ID NO: 37) *Homo sapiens* KIAA1274 (KIAA1274), mRNA, accession numbers NM_014431 and XM_166125; (SEQ ID NO: 38) *Homo sapiens* taste receptor, type 2, member 10 (TAS2R10), mRNA, accession number NM_023921; (SEQ ID NO: 39) *Homo sapiens* ribosomal protein S20 (RPS20), transcript variant 2, mRNA, accession number NM_001023; (SEQ ID NO: 40) Homo sapiens glycerol-3-phosphate acyltransferase 2, mitochondrial (GPAT2), nuclear gene encoding mitochondrial protein, mRNA, accession number NM_207328; (SEQ ID NO: *41) Homo sapiens* hypothetical protein LOC643008 (LOC643008), transcript variant 1, mRNA, accession numbers NM_001162995 and NR_024379; (SEQ ID NO: 42) *Homo sapiens* keratin associated protein 6-2 (KRTAP6-2), mRNA, accession number NM_181604; (SEQ ID NO: 43) *Homo sapiens* saitohin (STH), mRNA, accession number NM_001007532; (SEQ ID NO: 44) *Homo sapiens* olfactory receptor, family 2, subfamily A, member 2 (OR2A2), mRNA, accession number NM_001005480 and XM_498253; (SEQ ID NO: 45) *Homo sapiens* proteinase 3 (PRTN3), mRNA, accession number NM_002777; (SEQ ID NO: 46) *Homo sapiens* pregnancy specific beta-1-glycoprotein 9 (PSG9), mRNA, accession number NM_002784; (SEQ ID NO: 47) *Homo sapiens* guanylate cyclase activator 2B (uroguanylin) (GUCA2B), mRNA, accession number NM_007102; (SEQ ID NO: 48) *Homo sapiens* armadillo repeat containing 10 (ARMC10), transcript variant A, mRNA, accession number NM_031905; (SEQ ID NO: 49) *Homo sapiens* chromosome 11 open reading frame 59 (C11orf59), mRNA, accession number NM_017907; (SEQ ID NO: 50) Homo sapiens coiled-coil-helix-coiled-coil-helix domain containing 10, (CHCHD10), mRNA, accession number NM_213720; (SEQ ID NO: 51) *Homo sapiens* 2-oxoglutarate and iron-dependent oxygenase domain containing 2 (OGFOD2), mRNA, accession number NM_024623; (SEQ ID NO: 52) *Homo sapiens* biogenesis of lysosomal organelles complex-1, subunit 1 (BLOC1S1), mRNA, accession number NM_001487; (SEQ ID NO: 53) *Homo sapiens* apolipoprotein A-I (APOA1), mRNA, accession number NM_000039; (SEQ ID NO: 54) *Homo sapiens* CD3e molecule, epsilon (CD3-TCR complex) (CD3E), mRNA, accession number NM_000733; (SEQ ID NO: 55) *Homo sapiens* keratinocyte differentiation-associated protein (KRTDAP), mRNA, accession number NM_207392; (SEQ ID NO: 56) *Homo sapiens* PDZ domain containing 1 (PDZK1), mRNA, accession numbers NM_002614, XM_936907, XM_943050, XM_943061, and XM_943068; (SEQ ID NO: 57) *Homo sapiens* N-acetyltransferase 14 (GCN5-related, putative) (NAT14), mRNA, accession number NM_020378; (SEQ ID NO: 58) *Homo sapiens* keratin 17 (KRT17), mRNA, accession number NM_000422; (SEQ ID NO: 59) *Homo sapiens* ribosomal protein S19 binding protein 1 (RPS19BP1), mRNA, accession numbers NM_194326 and XM_039373; (SEQ ID NO: 60) *Homo sapiens* transmembrane protein 188 (TMEM188), mRNA, accession number NM_153261; (SEQ ID NO: 61) *Homo sapiens* cysteine and glycine-rich protein 2 (CSRP2), mRNA, accession number NM_001321; (SEQ ID NO: 62) *Homo sapiens* olfactory receptor, family 4, subfamily D, member 1 (OR4D1), mRNA, accession numbers NM_012374 and XM_292627; (SEQ ID NO: 63) *Homo sapiens* ribosomal protein L8 (RPL8), transcript variant 1, mRNA, accession number NM_000973; (SEQ ID NO: 64) *Homo sapiens* tumor necrosis factor receptor superfamily, member 13C (TNFRSF13C), mRNA, accession number NM_052945; (SEQ ID NO: 65) *Homo sapiens* mitochondrial ribosomal protein S21 (MRPS21), nuclear gene encoding mitochondrial protein, transcript variant 2, mRNA, accession number NM_018997; (SEQ ID NO: 66) *Homo sapiens* apolipoprotein A-IV (APOA4), mRNA, accession number NM_000482; (SEQ ID NO: 67) *Homo sapiens* junctional sarcoplasmic reticulum protein 1 (JSRP1), mRNA, accession number NM_144616; (SEQ ID NO: 68) *Homo sapiens* proteasome (prosome, macropain) activator subunit 2 (PA28 beta) (PSME2), mRNA, accession number NM_002818; (SEQ ID NO: 69) *Homo sapiens* zinc finger and BTB domain containing 5 (ZBTB5), mRNA, accession number NM_014872 and XM_376832; (SEQ ID NO: 70) *Homo sapiens* chromosome 10 open reading frame 95, mRNA (cDNA clone MGC:161737 IMAGE:8992175), complete cds, accession number, BC126459; (SEQ ID NO: 71) *Homo sapiens* nicotinamide phosphoribosyltransferase (NAMPT), mRNA, accession number NM_005746; (SEQ ID NO: 72) *Homo sapiens* trace amine associated receptor 6 (TAAR6), mRNA, accession number, NM_175067; (SEQ ID NO: 73) *Homo sapiens* myosin, light chain 6, alkali, smooth muscle and non-muscle (MYL6), transcript variant 1, mRNA, accession numbers NM_021019 and NM_079424; (SEQ ID NO: 74) *Homo sapiens* ATP synthase, H+ transporting, mitochondrial Fo complex, subunit C2 (subunit 9) (ATP5G2), nuclear gene encoding mitochondrial protein, transcript variant 2, mRNA, accession number NM_005176; (SEQ ID NO: 75) *Homo sapiens* family with sequence similarity 18, member B2 (FAM18B2), transcript variant 1, mRNA, accession numbers NM_145301 and XM_936923; (SEQ ID NO: 76) *Homo sapiens* Sp6 transcription factor (SP6), mRNA, accession numbers NM_199262 and XM_292621; (SEQ ID NO: 77) *Homo sapiens* inverted formin, FH2 and WH2 domain containing (INF2), transcript variant 1, mRNA, accession number NM_022489; (SEQ ID NO: 78) *Homo sapiens* Rho GDP dissociation inhibitor (GDI) alpha (ARHGDIA), transcript variant 2, mRNA, accession number NM_004309; (SEQ ID NO: 79) *Homo sapiens* OTU domain containing 6A (OTUD6A), mRNA, accession number NM_207320; (SEQ ID NO: 80) *Homo sapiens* zinc finger and BTB domain containing 12 (ZBTB12), mRNA, accession number NM_181842; (SEQ ID NO: 81) *Homo sapiens* mitotic spindle organizing protein 2B (MZT2B), mRNA, accession number NM_025029; (SEQ ID NO: 82) *Homo sapiens* olfactory receptor, family 52, subfamily E, member 2 (OR52E2), mRNA, accession number NM_001005164 and XM_061610; (SEQ ID NO: 83) *Homo sapiens* hypothetical LOC150622 (LOC150622), non-coding RNA, accession number NR_026832, XR_041760, XR_041761, and XR_041762; (SEQ ID NO: 84) *Homo sapiens* selenophosphate synthetase 1 (SEPHS1), transcript variant 1, mRNA, accession number NM_012247; (SEQ ID NO: 85) *Homo sapiens* barrier to autointegration factor 1 (BANF1), transcript variant 1, mRNA, accession number NM_003860; (SEQ ID NO: 86) *Homo sapiens* general transcription factor IIB (GTF2B), mRNA, accession number NM_001514; (SEQ ID NO: 87) *Homo sapiens* RGM domain family, member A (RGMA), transcript variant 4, mRNA, accession number NM_020211; (SEQ ID NO: 88) *Homo sapiens* prolactin releasing hormone receptor (PRLHR), mRNA, accession number NM_004248 and NM_005287; (SEQ ID NO: 89) *Homo sapiens* dpy-19-like 2 pseudogene 2 (C. elegans) (DPY19L2P2), transcript variant 2, non-coding RNA, accession number NR_003561; (SEQ ID NO: 90) *Homo sapiens* meteorin, glial cell differentiation regulator (METRN), mRNA, accession number NM_024042; (SEQ ID NO: 91) *Homo sapiens* free fatty acid receptor 1 (FFAR1), mRNA, accession number NM_005303; (SEQ ID NO: 92) *Homo sapiens* natriuretic peptide B (NPPB), mRNA, accession number NM_002521; (SEQ ID NO: 93) *Homo sapiens* BCL2/adenovirus E1B 19kDa interacting protein 3 (BNIP3), nuclear gene encoding mitochondrial protein, mRNA, accession number NM_004052; (SEQ ID NO: 94) *Homo sapiens* basic helix-loop-helix family, member a15 (BHLHA15), mRNA, accession number NM_177455; (SEQ ID NO: 95) *Homo sapiens* Finkel-Biskis-Reilly murine sarcoma virus (FBR-MuSV) ubiquitously expressed (FAU), mRNA, accession number NM_001997; (SEQ ID NO: 96) *Homo sapiens* chromosome 9 open reading frame 70 (C9orf70), non-coding RNA, accession number NR_026663 and XM_001721481 XM_001723928 XM_001724353; (SEQ ID NO: 97) *Homo sapiens* ribosomal protein L30 (RPL30), mRNA, accession number NM_000989; (SEQ ID NO: 98) *Homo sapiens* meteorin, glial cell differentiation regulator-like (METRNL), mRNA, accession number NM_001004431 and XM_209073; (SEQ ID NO: 99) *Homo sapiens* ubiquitin-like 5 (UBL5), transcript variant 1, mRNA, accession number NM_024292; (SEQ ID NO: 100) *Homo sapiens* potassium inwardly-rectifying channel, subfamily J, member 4, (KCNJ4), transcript variant 1, mRNA, accession number NM_152868; (SEQ ID NO: 101) *Homo sapiens* nascent polypeptide-associated complex alpha subunit (NACA), transcript variant 1, mRNA, accession number NM_001113203; (SEQ ID NO: 102) *Homo sapiens* small EDRK-rich factor 2 (SERF2), mRNA, accession number NM_001018108; (SEQ ID NO: 103) *Homo sapiens* sulfotransferase family, cytosolic, 1A, phenol-preferring, member 2 (SULT1A2), transcript variant 2, mRNA, accession number NM_177528; (SEQ ID NO: 104) *Homo sapiens* olfactory receptor, family 51, subfamily G, member 2 (OR51G2), mRNA, accession number NM_001005238; (SEQ ID NO: 105) *Homo sapiens* basic transcription factor 3 (BTF3), transcript variant 1, mRNA, accession number NM_001037637; and (SEQ ID NO: 106) *Homo sapiens* LSM10, U7 small nuclear RNA associated (LSM10), mRNA, accession number NM_032881.

In one embodiment, the CFP RNA PTB biomarkers can include a biomarker that is up-regulated in order to indicate susceptibility to PTB having SEQ ID NOs: 107-142, wherein the Probset ID, accession numbers, Gene Symbols, and start and stop of the sequences thereof are incorporated herein by specific reference:

| **SEQ ID NO:** | **#** | **Probeset ID** | **Gene Symbol** | **RefSeq(Accession)** | **Seqname** | **Start** | **Stop** |
|---|---|---|---|---|---|---|---|
| SEQ ID NO: | **107** | 2391026 | **C1orf159** | **BC008788** | chr1 | 1020631 | 1020674 |
| SEQ ID NO: | **108** | 2465373 | **AHCTF1** | NM_015446 | chr1 | 247063497 | 247063521 |
| SEQ ID NO: | **109** | 2321026 | **C1orf158** | NM_152290 | chr1 | 12821062 | 12821086 |
| SEQ ID NO: | **110** | 2370564 | **CACNA1E** | NM_000721 | chr1 | 181705411 | 181705435 |
| SEQ ID NO: | **111** | 2445415 | **ASTN1** | NM_004319 | chr1 | 176927594 | 176927618 |
| SEQ ID NO: | **112** | 2383404 | **ADCK3** | NM_020247 | chr1 | 227165195 | 227165219 |
| SEQ ID NO: | **113** | 3314648 | **C10orf92** | BC034223 | chr10 | 134628211 | 134628236 |
| SEQ ID NO: | **114** | 3332991 | **C11orf66** | NM_145017 | chr11 | 61257978 | 61258037 |
| SEQ ID NO: | **115** | 3377201 | **CDC42BPG** | NM_017525 | chr11 | 64601758 | 64601797 |
| SEQ ID NO: | **116** | 3381279 | **ARAP1** | BC056401 | chr11 | 72411090 | 72411124 |
| SEQ ID NO: | **117** | 3403055 | **ATN1** | NM_001007026 | chr12 | 7045236 | 7045261 |
| SEQ ID NO: | **118** | 3655114 | **CD19** | NM_001178098 | chr16 | 28943903 | 28943933 |
| SEQ ID NO: | **119** | 3739970 | ABR | NM_021962 | chr17 | 913969 | 913994 |
| SEQ ID NO: | **120** | 3774020 | **C17orf70** | NR_033338 | chr17 | 79518798 | 79518881 |
| SEQ ID NO: | **121** | 3774725 | **CCDC57** | ENST00000324808 | chr17 | 80109446 | 80109470 |
| SEQ ID NO: | **122** | 3741735 | **CAMKK1** | AF370377 | chr17 | 3773035 | 3773059 |
| SEQ ID NO: | **123** | 3830886 | **ARHGAP33** | NM_052948 | chr19 | 36273687 | 36273769 |
| SEQ ID NO: | **124** | 3835887 | **APOE** | NM_000041 | chr19 | 45412388 | 45412412 |
| SEQ ID NO: | **125** | 3866306 | **AP2S1** | NM_004069 | chr19 | 47342008 | 47342032 |
| SEQ ID NO: | **126** | 2546857 | **CAPN13** | NM_144575 | chr2 | 30993219 | 30993282 |
| SEQ ID NO: | **127** | 2576644 | **C2orf27B** | BC043584 | chr2 | 132552867 | 132552917 |
| EQ ID NO: | **128** | 2546826 | **CAPN13** | NM_144575 | chr2 | 30961299 ....., | 30961323 |
| SEQ ID NO: | **129** | 2708707 | **C3orf70** | NM_001025266 | chr3 | 184870647 ^{...,} | 184870677 |
| SEQ ID NO: | **130** | 2622179 | **BSN** | NM_003458 | chr3 | 49699843 | 49699867 |
| SEQ ID NO: | **131** | 2870730 | **BCLAF1** | NM_014739 | chr5 | 110285528 | 110285604 |
| SEQ ID NO: | **132** | 2902959 | **C4A** | ENST00000428956 | chr6 | 31949811 | 31949835 |
| SEQ ID NO: | **133** | 2953468 | **C6orf130** | ENST00000488238 | chr6 | 41043021 | 41043070 |
| SEQ ID NO: | **134** | 3031798 | **ABCB8** | NM_007188 | chr7 | 150744493 | 150744517 |
| SEQ ID NO: | **135** | 3039763 | **ANKMY2** | NM_020319 | chr7 | 16666684 | 16666799 |
| SEQ ID NO: | **136** | 3023426 | **AHCYL2** | NM_001130723 | chr7 | 129008311 | 129008335 |
| SEQ ID NO: | **137** | 3031951 | **AGAP3** | NM_031946 | chr7 | 150841064 | 150841088 |
| SEQ ID NO: | **138** | 3031944 | **AGAP3** | AL442089 | chr7 | 150838958 | 150838982 |
| SEQ ID NO: | **139** | 3206269 | **ATP5A1** | NM_001001937 | chr9 | 41799773 | 41799797 |
| SEQ ID NO: | **140** | 4001353 | **BEND2** | NM_153346 | chrX | 18221855 | 18222031 |
| SEQ ID NO: | **141** | 3969900 | **CA5B** | ENST00000479740 | chrX | 15768063 | 15768093 |
| SEQ ID NO: | **142** | 3966810 | **CD99P1** | NR_033380 | chrX | 2541426 | 2541450 |

In one embodiment, the CFP RNA PTB biomarkers can include a biomarker that is down-regulated in order to indicate susceptibility to PTB.

| SEO ID NO | # | Probeset ID | Gene Symbol | RefSeq (Accession) | Seqname | start | stop |
|---|---|---|---|---|---|---|---|
| SEQ ID NO | 143 | 2434348 | APH1A | AK125685 | chr1 | 150239436 | 150239470 |
| SEQ ID NO: | 144 | 2347527 | ABCD3 | NM_001122674 | chr1 | 94944215 | 94944239 |
| SEO ID NO: | 145 | 2347504 | ABCD3 | NM_002858 | chr1 | 194884035 | 94884129 |
| SEQ ID NO: | 146 | 2347024 | CCDC18 | NM_206886 | chr1 | 193645587 | 93645967 |
| SEO ID NO: | 147 | 2383766 | ARF1 | NM_001024227 | chr1 | 228286406 | 228286440 |
| SEQ ID NO: | 148 | 2411671 | AGBL4 | ENST00000411952 | chur1 | 49052585 | 49052609 |
| SEQ ID NO: | 149 | 2316059 | ATAD3A | NM_018188 | chur1 | 1469347 | 1469376 |
| SEQ ID NO: | 150 | 2391349 | ACAP3 | AB051503 | chur1 | 1240376 | 1240469 |
| SEQ ID NO: | 151 | 2385702 | C1orf57 | NM_032324 | chur1 | 233086457 | 233086481 |
| SEQ ID NO: | 152 | 2393833 | C1orf174 | NM_207356 | chr1 | 3816811 | 3816835 |
| SEQ ID NO: | 153 | 2440696 | B4GALT3 | NM_003779 | chr1 | 161147290 | 161147314 |
| SEQ ID NO: | 154 | 2383363 | ADCK3 | ENST00000366779 | chr1 | 227096295 | 227096344 |
| SEQ ID NO: | 155 | 2318458 | CAMTA1 | NM_015215 | chr1 | 6845541 | 6845635 |
| SEQ ID NO: | 156 | 2392132 | C1orf86 | ENST00000378545 | chr1 | 2130199 | 2130245 |
| SEQ ID NO: | 157 | 2399312 | ALDH4A1 | NM_003748 | chr1 | 19199312 | 19199336 |
| SEQ ID NO: | 158 | 3286039 | CCNYL2 | ENST00000345581 | chr10 | 42965620 | 42965646 |
| SEQ ID NO: | 159 | 3282296 | ACBD5 | ENST00000375888 | chr10 | 27529434 | 27529579 |
| SEQ ID NO: | 160 | 3261217 | BTRC | NM_033637 | chr10 | 103285926 | 103285955 |
| SEQ ID NO: | 161 | 3284165 | C1D | NM_173177 | chr10 | 32800666 | 32800698 |
| SEQ ID NO: | 162 | 3245187 | ANXA8L2 | NM_001630 | chr10 | 47747028 | 47747107 |
| SEQ ID NO: | 163 | 3251356 | ANAPC16 | NM_173473 | chr10 | 73975872 | 73975896 |
| SEQ ID NO: | 164 | 3380994 | C11orf59 | NM_017907 | chr11 | 71814234 | 71814263 |
| SEQ ID NO: | 165 | 3353451 | C11orf63 | NM_024806 | chr11 | 122774660 | 122774979 |
| SEQ ID NO: | 166 | 3370889 | ALX4 | NM_021926 | chr11 | 44296901 | 44297069 |
| SEQ ID NO: | 167 | 3364964 | ABCC8 | NM_000352 | chr11 | 17453766 | 17453791 |
| SEQ ID NO: | 168 | 3377365 | BATF2 | NM_138456 | chr11 | 64757241 | 64757266 |
| SEQ ID NO: | 169 | 3351287 | CD3E | NM_000733 | chr11 | 118175668 | 118175692 |
| SEQ ID NO: | 170 | 3323765 | ANO5 | NM_213599 | chr11 | 22215039 | 22215069 |
| SEQ ID NO: | 171 | 3352074 | CBL | NM_005188 | chr11 | 119077128 | 119077154 |
| SEQ ID NO: | 172 | 3332702 | CD6 | NM_006725 | chr11 | 60785827 | 60785851 |
| SEQ ID NO: | 173 | 3378518 | C1QBP | NM_001212 | chr11 | 66529422 | 66529450 |
| SEQ ID NO: | 174 | 3334998 | CAPN1 | NM_005186 | chr11 | 64978760 | 64978949 |
| SEQ ID NO: | 175 | 3316546 | AP2A2 | NM_012305 | chr11 | 1010548 | 1010572 |
| SEQ ID NO: | 176 | 3358122 | C11orf35 | NM_173573 | chr11 | 556268 | 556374 |
| SEQ ID NO: | 177 | 3457551 | ANKRD52 | NM_173595 | chr12 | 56631722 | 56631752 |
| SEQ ID NO: | 178 | 3440081 | CACNA2D4 | NM_172364 | chr12 | 1909167 | 1909199 |
| SEQ ID NO: | 179 | 3431564 | C12orf24 | AK297684 | chr12 | 110924538^{.} | 110924563 |
| SEQ ID NO: | 180 | 3434501 | CABP1 | NM_031205 | chr12 | 121088358 | 121088431 |
| SEQ ID NO: | 181 | 3435685 | ARL61P4 | NM_018694 | chr12 | 123466154 | 123466179 |
| SEQ ID NO: | 182 | 3413611 | CACNB3 | NM_000725 | chr12 | 49212713 | 49212756 |
| SEQ ID NO: | 183 | 3523859 | C13orf27 | NM_138779 | chr13 | 103418821 | 103418856 |
| SEQ ID NO: | 184 | 3573232 | ALKBH1 | NM_006020 | chr14 | 78140155 | 78140183 |
| SEQ ID NO: | 185 | 3563711 | C14orf138 | NM_024558 | chr14 | 50583243 | 50583268 |
| SEQ ID NO: | 186 | 3543628 | C14orf169 | NM_024644 | chr14 | 73957998 | 73958031 |
| SEQ ID NO: | 187 | 3576909 | ATXN3 | NR_028453 | chr14 | 92547321 | 92547345 |
| SEQ ID NO: | 188 | 3557166 | ACIN1 | NM_014977 | chr14 | 23564322 | 23564348 |
| SEQ ID NO: | 189 | 3604597 | ADAMTS7 | NM_014272 | chr15 | 82611989 | 82612090 |
| SEQ ID NO: | 190 | 3601544 | CCDC33 | NM_025055 | chr15 | 74536429 | 74536486 |
| SEQ ID NO: | 191 | 3605931 | ALPK3 | NM_020778 | chr15 | 85411431 | 85411647 |
| SEQ ID NO: | 192 | 3619410 | C15orf52 | NM_207380 | chr15 | 40627985 | 40628027 |
| SEQ ID NO: | 193 | 3605398 | ADAMTSL3 | NM_207517 | chr15 | 84324481 | 84324513 |
| SEQ ID NO: | 194 | 3636496 | BTBD1 | NM_025238 | chr15 | 83735879 | 83735903 |
| SEQ ID NO: | 195 | 3628544 | CA12 | NM_001218 | chr15 | 63637686 | 63637806 |
| SEQ ID NO: | 196 | 3601237 | CD276 | NM_001024736 | chr15 | 73992032 | 73992056 |
| SEQ ID NO: | 197 | 3607736 | C15orf42 | NM_152259 | chr15 | 90167064 | 90167094 |
| SEQ ID NO: | 198 | 3620776 | CDAN1 | NM_138477 | chr15 | 43026443 | 43026535 |
| SEQ ID NO: | 199 | 3619407 | C15orf52 | NM_207380 | chr15 | 40627389 | 40627585 |
| SEQ ID NO: | 200 | 3617732 | ACT1 | NM_005159 | chr15 | 35084610 | 35084634 |
| SEQ ID NO: | 201 | 3619427 | C15orf52 | AK126485 | chr15 | 40631674 | 40631701 |
| SEQ ID NO: | 202 | 3655082 | ATP2A1 | NM_173201 | chr16 | 28909568 | 28909754 |
| SEQ ID NO: | 203 | 3656848 | BCKDK | NM_001122957 | chr16 | 31123381 | 31123415 |
| SEQ ID NO: | 204 | 3695322 | CDH16 | NM_004062 | chr16 | 66944302 | 66944327 |
| SEQ ID NO: | 205 | 3704743 | ANKRD11 | NM_013275 | chr16 | 89351849 | 89352020 |
| SEQ ID NO: | 206 | 3662891 | CCDC135 | NM_032269 | chr16 | 57738788 | 57738820 |
| SEQ ID NO: | 207 | 3687096 | BOLA2 | NM_001031827 | chr16 | 30204743 | 30204770 |
| SEQ ID NO: | 208 | 3686627 | APOB48R | NM_018690 | chr16 | 28507548 | 28507572 |
| SEQ ID NO: | 209 | 3770812 | CASKIN2 | NM_020753 | chr17 | 73499499 | 73499557 |
| SEQ ID NO: | 210 | 3767486 | AXIN2 | NM_004655 | chr17 | 63533032 | 63533167 |
| SEQ ID NO: | 211 | 3742217 | ALOX15 | NM_001140 | chr17 | 4535481 | 4535558 |
| SEQ ID NO: | 212 | 3742483 | CAMTA2 | NM_015099 | chr17 | 4876890 | 4877042 |
| SEQ ID NO: | 213 | 3764300 | BZRAP1 | BX648763 | chr17 | 56382268 | 56382298 |
| SEQ ID NO: | 214 | 3722682 | C17orf88 | NK_026770 | chr17 | 41994608 | 41994632 |
| SEQ ID NO: | 215 | 3766544 | CDd79B | NM_000626 | chr17 | 62008702 | 62008726 |
| SEQ ID NO: | 216 | 3748962 | ALDH3A1 | NM_001135168 | chr17 | 19641470 | 19641494 |
| SEQ ID NO: | 217 | 3774985 | C17orf101 | NR_033265 | chr17 | 80350291 | 80350395 |
| SEQ ID NO: | 218 | 3764359 | BZRAP1 | NM_004758 | chr17 | 56404109 | 56404137 |
| SEQ ID NO: | 219 | 3774706 | CCDC57 | NM_198082 | chr17 | 80059693 | 80059731 |
| SEQ ID NO: | 220 | 3773685 | AZI1 | NM_014984 | chr17 | 79172707 | 79172736 |
| SEQ ID NO: | 221 | 3773633 | AATK | ENST00000417379 | chr17 | 79105718 | 79105746 |
| SEQ ID NO: | 222 | 3848059 | C3 | NM_000064 | chr19 | 6684786 | 6684810 |
| SEQ ID NO: | 223 | 3866980 | CARD8 | NM_001184900 | chr19 | 48715191 | 48715220 |
| SEQ ID NO: | 224 | 3850462 | AP1M2 | NM_005498 | chr19 | 10685580 | 10685611 |
| SEQ ID NO: | 225 | 3854387 | ANO8 | NM_020959 | chr19 | 17439225 | 17439281 |
| SEQ ID NO: | 226 | 3837683 | C19orf68 | BC043386 | chr19 | 48700487 | 48700516 |
| SEQ ID NO: | 227 | 3867278 | CA11 | NM_001217 | chr19 | 49143358 | 49143452 |
| SEQ ID NO: | 228 | 3865986 | CCDC8 | NM_032040 | chr19 | 46916087 | 46916262 |
| SEQ ID NO: | 229 | 3846260 | C19orf28 | NM_021731 | chr19 | 3557104 | 3557128 |
| SEQ ID NO: | 230 | 3868520 | ASPDH | NM_001114598 | chr19 | 51014987 | 51015030 |
| SEQ ID NO: | 231 | 3860221 | ALKBH6 | NM_198867 | chr19 | 36502307 | 36502333 |
| SEQ ID NO: | 232 | 3815214 | AZU1 | NM_001700 | chr19 | 828320 | 82837371 |
| SEQ ID NO: | 233 | 3817205 | ATCAY | NM_033064 | chr19 | 3917745 | 3917775 |
| SEQ ID NO: | 234 | 3846377 | APBA3 | NM_004886 | chr19 | 3754199 | 3754228 |
| SEQ ID NO: | 235 | 3830369 | CD22 | NM_001771 | chr19 | 35823497 | 35823523 |
| SEQ ID NO: | 236 | 3842076 | BRSK1 | NM_032430 | chr19 | 55805471 | 5805501 |
| SEQ ID NO: | 237 | 3852137 | CACNA1A | NM_000068 | chr19 | 13318294 | 13318431 |
| SEQ ID NO: | 238 | 3824734 | ARRDC2 | NM_015683 | chr19 | 18121452 | 18121479 |
| SEQ ID NO: | 239 | 3834055 | AXL | NM_021913 | chr19 | 41737096 | 41737140 |
| SEQ ID NO: | 240 | 3846299 | C19orf29 | NM_001080543 | chr19 | 3613163 | 3613278 |
| SEQ ID NO: | 241 | 3836141 | BLOC1S3 | NM_212550 | chr19 | 45683121 | 45683155 |
| SEQ ID NO: | 242 | 3832352 | CATSPERG | NM_021185 | chr19 | 38852853 | 38852886 |
| SEQ ID NO: | 243 | 3843980 | A1BG-AS | BC040926 | chr19 | 58864701 | 58864725 |
| SEQ ID NO: | 244 | 3846310 | C19orf29 | NM_001080543 | chr19 | 3620730 | 3620754 |
| SEQ ID NO: | 245 | 3839117 | ATF5 | NM_012068 | chr19 | 50436321 | 50436349 |
| SEQ ID NO: | 246 | 2521240 | CCDC150 | NM_001080539 | chr2 | 197504344 | 197504405 |
| SEQ ID NO: | 247 | 2474325 | C2orf28 | NM_016085 | chr2 | 27435237 | 27435334 |
| SEQ ID NO: | 248 | 2574650 | BIN1 | NM_139343 | chr2 | 127808045 | 127808098 |
| SEQ ID NO: | 249 | 2473975 | C2orf18 | NM_017877 | chr2 | 27001867 | 27001894 |
| SEQ ID NO: | 250 | 2500292 | BCL2L11 | AB071199 | chr2 | 111887709 | 111887791 |
| SEQ ID NO: | 251 | 2505925 | ARHGEF4 | NM_015320 | chr2 | 131804327 | 131804358 |
| SEQ ID NO: | 252 | 2532302 | ALPPL2 | NM_031313 | chr2 | 233272604 | 233272635 |
| SEQ ID NO: | 253 | 2536644 | BOK | NM_032515 | chr2 | 242512472 | 242512497 |
| SEQ ID NO: | 254 | 2566556 | C2orf55 | NM_207362 | chr2 | 99454585 | 99454610 |
| SEQ ID NO: | 255 | 2532289 | ALPP | NM_001632 | chr2 | 233246242 | 233246266 |
| SEQ ID NO: | 256 | 2604401 | ARL4C | NM_005737 | chr2 | 235404210 | 235404234 |
| SEQ ID NO: | 257 | 3874441 | CDC25B | NM_021873 | chr20 | 3776391 | 3776523 |
| SEQ ID NO: | 258 | 3882227 | BPIL3 | NM_174897 | chr20 | 31625440 | 31625473 |
| SEQ ID NO: | 259 | 3894422 | ANGPT4 | NM_015985 | chr20 | 865725 | 865751 |
| SEQ ID NO: | 260 | 3874383 | ATRN | NM_139321 | chr20 | 3614963 | 3615036 |
| SEQ ID NO: | 261 | 3892803 | C20orf200 | NR_033263 | chr20 | 61142540 | 61142567 |
| SEQ ID NO: | 262 | 3914081 | ARFRP1 | NM_001134758 | chr20 | 62331883 | 62331908 |
| SEQ ID NO: | 263 | 3882563 | CBFA2T2 | NM_005093 | chr20 | 32194762 | 32194787 |
| SEQ ID NO: | 264 | 3907034 | ADA | NM_000022 | chr20 | 43280223 | 43280248 |
| SEQ ID NO: | 265 | 3926166 | C21orf91 | ENST00000405964 | chr21 | 19191195 | 19191284 |
| SEQ ID NO: | 266 | 3932407 | C21orf88 | NR_026542 | chr21 | 40984265 | 40984292 |
| SEQ ID NO: | 267 | 3922457 | ABCG1 | NM_016818 | chr21 | 43639267 | 43639291 |
| SEQ ID NO: | 268 | 3918143 | C21orf63 | AK126660 | chr21 | 33829548 | 33829572 |
| SEQ ID NO: | 269 | 3951118 | ACR | ENST00000216139 | chr22 | 51176638 | 51176663 |
| SEQ ID NO: | 270 | 3946042 | CACNA1l | NM_021096 | chr22 | 40081973 | 40082331 |
| SEQ ID NO: | 271 | 3955347 | C22orf13 | ENST00000407973 | chr22 | 24951587 | 24951829 |
| SEQ ID NO: | 272 | 2644874 | BPESC1 | NR_026783 | chr3 | 138824138 | 138824168 |
| SEQ ID NO: | 273 | 2641457 | CCDC48 | NM_024768 | chr3 | 128751742 | 128751767 |
| SEQ ID NO: | 274 | 2627379 | C3orf49 | NR_026866 | chr3 | 63830699 | 63830723 |
| SEQ ID NO: | 275 | 2624738 | CACNA2D3 | NM_018398 | chr3 | 54913057 | 54913081 |
| SEQ ID NO: | 276 | 2681152 | C3orf64 | AK304102 | chr3 | 69062765 | 69062816 |
| SEQ ID NO: | 277 | 2687780 | CD47 | NM_001777 | chr3 | 107769425 | 107769449 |
| SEQ ID NO: | 278 | 2719502 | CC2D2A | NM_001080522 | chr4 | 15504114 | 15504140 |
| SEQ ID NO: | 279 | 2852783 | C1QTNF3 | NM_030945 | chr5 | 34033484 | 34033517 |
| SEQ ID NO: | 280 | 2881766 | ANXA6 | NM_001155 | chr5 | 150496698 | 150496722 |
| SEQ ID NO: | 281 | 2842463 | C5orf25 | AK126204 | chr5 | 175721931 | 175722032 |
| SEQ ID NO: | 282 | 2878396 | APBB3 | AK125244 | chr5 | 139943698 | 139943736 |
| SEQ ID NO: | 283 | 4047621 | BTNL8 | NM_024850 | chr5 | 180375920 | 180375946 |
| SEQ ID NO: | 284 | 2901692 | ABCF1 | NM_001025091 | chr6 3 | 30545599 | 30545665 |
| SEQ ID NO: | 285 | 2973284 | C6orf174 | NM_001012279 | chr61 | 127837554 | 127837578 |
| SEQ ID NO: | 286 | 2937603 | C6orf70 | NM_018341 | chr6 | 170175406 | 170175442 |
| SEQ ID NO: | 287 | 2999777 | AEBP1 | NM_001129 | chr7 | 44148891 | 44148940 |
| SEQ ID NO: | 288 | 3001005 | ABCA13 | NM_152701 | chr7 | 18285460 | 48285484 |
| SEQ ID NO: | 289 | 3017084 | ARMC10 | NM_031905 | chr7 | 102716226 | 102716250 |
| SEQ ID NO: | 290 | 3006668 | AUTS2 | NM_015570 | chr7 | 69599533 | 69599557 |
| SEQ ID NO: | 291 | 3158462 | C8ORFK29 | NR_015428 | chr8 | 145577092 | 145577180 |
| SEQ ID NO: | 292 | 3121027 | C8orf33 | NM_023080 | chr8 | 146278059 | 146278089 |
| SEQ ID NO: | 293 | 3105606 | CA2 | ENST00000285379 | chr8 | 86376123 | 86376148 |
| SEQ ID NO: | 294 | 3204670 | CD72 | ENST00000396759 | chr9 | 35618756 | 35618361 |
| SEQ ID NO: | 295 | 3221925 | AKNA | NM_030767 | chr9 | 117103978 | 117104002 |
| SEQ ID NO: | 296 | 3223849 | C5 | NM_001735 | chr9 | 123812463 | 123812487 |
| SEQ ID NO: | 297 | 3190991 | C9orf106 | NM_001012715 | chr9 | 132083295 | 132083325 |
| SEQ ID NO: | 298 | 3222599 | ASTN2 | NM_198186 | chr9 | 119449350 | 119449382 |
| SEQ ID NO: | 299 | 3229062 | BRD3 | NM_007371 | chr9 | 136905156 | 136905186 |
| SEQ ID NO: | 300 | 3986675 | ATG4A | ENST00000457035 | chrx | 107335082 | 107335109 |

An investigation was conducted to determine whether or not specific miRNA could be PTB biomarkers. The same total RNA extracted from the 26-28 week samples, described above, were run on the Affymetrix GeneChip non-coding small RNA array (e.g., 847 human non-coding small RNAs including miRNA, siRNA, snRNA, snoRNA, etc), and only miRNA altered in women destined for PTB were identified by fold change (e.g., ≥1.5x) and p value from control (p<0.01). The miRNA were ordered by narrowness of distribution (e.g., Affymetrix miRNA QC Tool and Ingenuity Systems Pathway Analysis). Of the 847 non-coding small RNA, only 14 were altered at 26 weeks in women destined for PTB; 3 CFP miRNA increased or were up-regulated (e.g., miRNA-548L (SEQ ID NO: 5), miRNA-99a (SEQ ID NO: 6), and miRNA-99b (SEQ ID NO: 7)); and 10 CFP miRNA decreased or were down-regulated (e.g., miRNA-382 (SEQ ID NO:8), miRNA-491 (SEQ ID NO: 9), miNRA-214 (SEQ ID NO: 10), miRNA-31 (SEQ ID NO: 11), miRNA-342 (SEQ ID NO: 12), miRNA-let-7g (SEQ ID NO: 13), miRNA-194-1 (SEQ ID NO: 14), miRNA-194-2 (SEQ ID NO: 15), miRNA 92b (SEQ ID NO: 16), miRNA 320b-1 (SEQ ID NO: 17), and miRNA 320b-2 (SEQ ID NO: 18). Genomic mapping revealed the PTB marker miRNAs were associated with cell regulation, muscle dysfunction, contractility and inflammation.

None are previously described in pregnancy and only a few previously associated with reproductive tissues. As miRNA reduce transcription and/or translation and 11 of 14 affected miRNAs are reduced, the findings may explain the activation process of myometrial activation which must precede PTB. That the pattern of miRNA change varied among PTB women suggests the patterns may reflect the underlying mechanism that causes PTB.

In one embodiment, the CFP miRNA PTB biomarkers can include a biomarker that increases in order to indicate susceptibility to PTB. These increasing CFP miRNA PTB biomarkers can include: miRNA-548L (SEQ ID NO: 5), see accession number NR_031630; miRNA-99a (SEQ ID NO: 6), see accession number NR_029514; and miRNA-99b (SEQ ID NO: 7), see accession number NR-029843.

In one embodiment, the CFP miRNA PTB biomarkers can include biomarker that decrease in order to indicate susceptibility to PTB. These decreasing CFP miRNA PTB biomarkers can include: miRNA-382 (SEQ ID NO:8), accession number NR_029874; miRNA-491 (SEQ ID NO: 9), accession number NR_030166; miNRA-214 (SEQ ID NO: 10), accession number NR_029627; miRNA-31 (SEQ ID NO: 11), accession number NR_029505; miRNA-342 (SEQ ID NO: 12), accession number NR_029888; miRNA-let-7g (SEQ ID NO: 13), accession number NR_029660; miRNA-194-1 (SEQ ID NO: 14), accession number NR_029711; miRNA-194-2 (SEQ ID NO: 15), accession number NR_029829; miRNA 92b (SEQ ID NO: 16), accession number NR_030281; miRNA 320b-1 (SEQ ID NO: 17), accession number NR_031564; and miRNA 320b-2 (SEQ ID NO: 18), accession number NR_0315748.

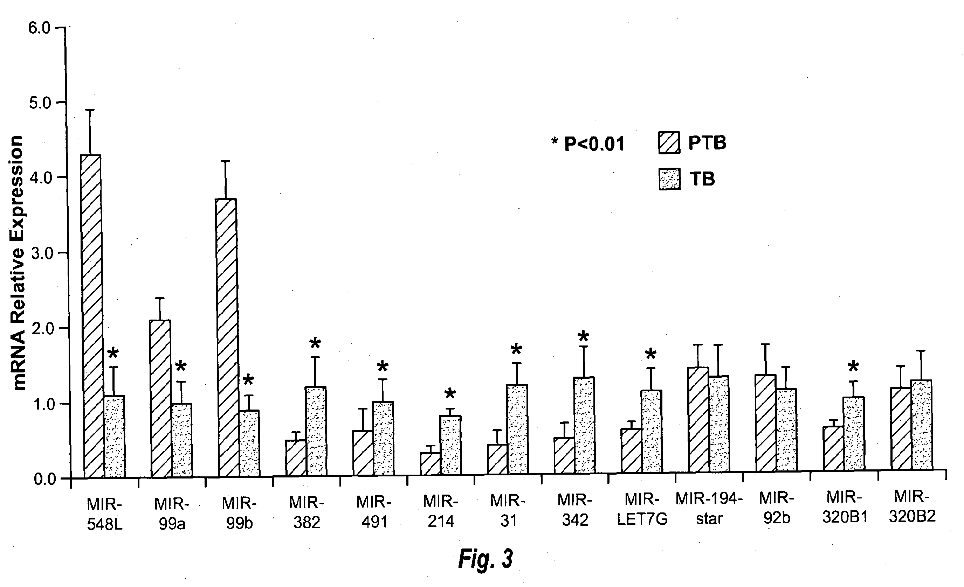

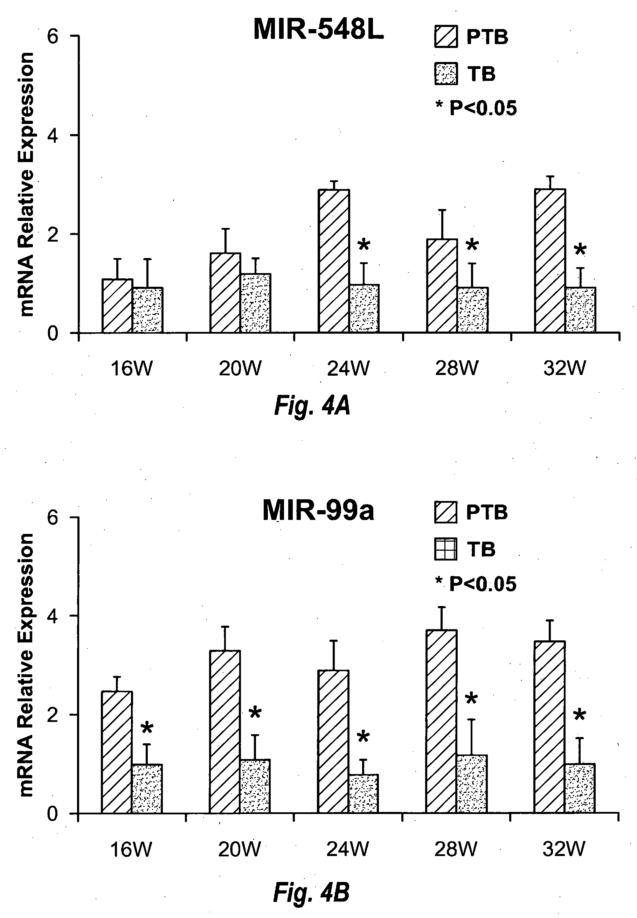

An investigation was also conducted to determine the pattern of miRNA that are altered as early as at 16 weeks in women destined for PTB. Validation of the array results was conducted by high-through put Real-time PCR. Briefly, 3 miRNA that were increased, 7 miRNA that were decreased, respectively at 26 weeks in women destined for spontaneous PTB, and Q-rtPCR was conducted and the miRNA were normalized with the normalization sequences described herein. Figure 3 confirms the 10/14 of miRNA array findings were significant altered in the miRNA PTB biomarker cell free plasma levels at 26 weeks in women destined for PTB. We also found that gestational age impact on CFP miRNA level. PCR studies were expanded to all biweekly samples available for these same pregnancies, and the PCR were normalized result with the normalization sequences (e.g., miRNA normalization sequence). Figure 4A indicates that the levels of miRNA-548L is altered only in early 2^{nd} trimester, and Figure 4B illustrates that miRNA-99a are actually significantly increased by 16 weeks gestation raising the possibility of a late 1^{st} testing window. This indicates that testing can be as early as 12 weeks, 10 weeks, and possibly even earlier. That is, the diagnostic testing can be implemented as early as the CFP RNA PTB biomarkers are modulated within the pregnant woman.

To simultaneously complete the validation of the about 296 CFP RNA PTB biomarkers and quantitate their levels across gestation, a PCR card was designed with custom designed primers to amplify the CFP miRNA PTB biomarkers and miRNA normalization sequences (e.g., an Applied Biosciences Taqman card preloaded with custom designed primers for the identified CFP miRNA PTB biomarkers and normalization miRNA sequences, wherein the primers can be readily determined from the sequences of the sequence listing by convention techniques, and may encompass low stringency, medium stringency and high stringency primers, and thereby the primer sequences that are useful can be changed within the sequences provided in the Sequence Listing). This PCR card utilizes high throughput microfluidic technology and allows for up to 384 Q-rtPCR wells with custom designed nested primers such as the CFP miRNA PTB biomarkers and normalization sequences. It is assumed commercialization will lead to the manufacture of large cards and the present invention is not limited to the existing dimensions. Each card requires only 50 ng of total miRNA. The cards were designed to accommodate multiple samples. Isolated CFP RNA was then applied to the custom PTB miRNA card in order to validate the miRNA array. That result is shown in Figure 3. With a sample size of 6 per group, the microarray results were validated for 10 of the 14 miRNA PTB markers. The miRNA symbols are shown as in Figure 3.

In one embodiment, the present invention includes a method of determining a primer or a probe for a CFP RNA PTB biomarker. Such a method can include analyzing one or more of the sequences of the Sequence Listing having SEQ ID NO: 5-300 and determining a unique or sufficiently unique specific target sequence that is useful as a primer or a probe therefore. The primers can be readily determined from the sequences of the sequence listing by convention techniques, and may encompass low stringency, medium stringency and high stringency primers, and thereby the primer sequences that are useful can be changed within the sequences provided in the Sequence Listing

While all of these CFP RNA PTB biomarkers are from humans, other biomarkers from other animals may also be found and used in veterinary practices.

In one embodiment, the CFP RNA PTB biomarkers can be used to predict whether or not a woman is destined for or susceptible to PTB. This determination can be performed by a blood test at least as early as 12 or 16 weeks gestation. Also, this same process can be applied to women with a multiple gestation with same markers. However, a newly derived set of unique markers applicable only to twins may be identified. Accordingly, the CFP RNA biomarkers identified herein can be combined in a mathematical algorithm that can predict likelihood of preterm birth. As there appears to be multiple pathways that lead to preterm birth. The algorithm may also be used to determine the mechanism causing the PTB in a given woman. The mathematics to create the algorithm is well known and not proprietary. Such an algorithm for predicting PTB can be run on a computing system, and may be configured as software and/or or hardware. Data can be input into the computing system in order to operate and optimize the PTB prediction algorithm.

In one embodiment, the present invention can include a method for predicting PTB in a woman pregnant with one fetus. Such a method can include determining a change in the CFP RNA transcriptome of a pregnant mother, wherein the change is predictive of preterm birth by the pregnant mother. Such a prediction of PTB can include extracting and isolating RNA from a body fluid of the pregnant mother at less than 32 weeks (e.g., 26-28 weeks, or as low as 12 weeks) of pregnancy. The isolated RNA can be used for determining a change in the RNA amount (e.g., at least a fold change, such as ≥1.5x) in the CFP RNA transcriptome of the pregnant mother, wherein the change is predictive of preterm birth by the pregnant mother.

In one embodiment, the present invention provides a method for predicting preterm birth in a woman pregnant with twins. Such a method of predicting PTB of twins can include determining a change in the pregnant woman's CFP RNA transcriptome, where the change is predictive of preterm birth by the pregnant mother. This method can include extracting and isolating RNA from a body fluid of the pregnant mother at less than 32 weeks (e.g., 26-28 weeks, or as low as 12 weeks) of pregnancy. The isolated RNA can be used for determining a change (e.g., at least a fold change, such as ≥1.5x) in the CFP RNA transcriptome of the pregnant mother, wherein the change is predictive of preterm birth by the pregnant mother.

In one embodiment, the present invention can include a method for predicting a pregnancy disease state. Such a method can include determining a change in the CFP RNA transcriptome of a pregnant mother, wherein the change is predictive of a pregnancy disease state. The method can include extracting and isolating RNA from a body fluid of the pregnant mother at less than 32 weeks (e.g., 26-28 weeks, or as low as 12 weeks) of pregnancy. The isolated RNA can then be used for determining a change (e.g., at least a fold change, such as ≥1.5x) in the CFP RNA transcriptome of the pregnant mother, wherein the change is predictive of a pregnancy disease state. For example, the pregnancy disease state can be poor placentation, fetal growth restriction, preeclampsia, or fetal anomalies.

In one embodiment, a method for predicting preterm birth can be performed by using the CFP RNA PTB biomarkers. Such a method can include determining a change in a CFP RNA transcriptome of a pregnant mother, wherein the change is predictive of preterm birth by the pregnant mother. Also, the method can include extracting and isolating CFP RNA from a body fluid of a pregnant mother at less than 32 weeks (e.g., 26-28 weeks, or as low as 12 weeks) of pregnancy. The method can also include determining a change, such as at least a fold change (e.g., ≥1.5x), in the CFP RNA transcriptome of the pregnant mother. The change in the CFP RNA transcriptome is predictive of preterm birth by the pregnant mother. In one aspect, the pregnant mother can be selected to be pregnant at less than 32 weeks of pregnancy and lacking preterm, premature rupture of membranes.

In one aspect, the extracted RNA from the pregnant mother can be processed through a whole-transcript expression array. In another aspect, the method can include identifying one or more RNA sequences that are predictive of preterm birth. For example, the pregnant mother can have one or more altered levels of RNA sequences selected from CFP RNA PTB biomarker sequences that are associated with expression, cell growth, cell proliferation, cell cycle, cell death, and cellular assembly and organization. The CFP RNA can be any type of CFP RNA, such as miRNA or mRNA. The RNA can be associated with cell regulation, muscle dysfunction, contractility and inflammation, and/or can be associated with myometrial quiescence and/or activation, and/or associated with expression, cell growth, cell proliferation, cell cycle, cell death, and cellular assembly and organization.

In one embodiment, the present invention can include a method of predicting preterm birth before 32 weeks of pregnancy. Such a method can include obtaining data regarding levels of biomarkers and gestation age and optionally other health factors. The data can then be input into a machine, which can process the data by computing the data in a mathematic model having parameters of levels of markers and gestation age and optionally other health factors. Such computing can be used for determining patient specific risk to preterm birth. In this method, the mathematical model can include parameters related to change in a preterm birth RNA biomarker amount, whether becoming present, increasing, or decreasing. The preterm birth RNA biomarker can be any of the RNA PTB biomarkers as described herein.

In one embodiment, the present invention can include a method of inhibiting, preventing, or treating PTB. Such a method would reflect identification of the mechanism causing the PTB in the individual woman based on the profile of the predictive PTB markers. The method can include various drug screening protocols that can impact or regulate a particular PTB biomarker, where such regulation can result in a reduced onset of PTB. The method can include obtaining a substance that blocks a message from one of the PTB RNA described herein. This can include blocking a biological signal of a PTB small RNA, mRNA, non-coding RNA, and/or miRNA. Once obtained, the substance can be administering to a pregnant woman prior to 32 weeks of pregnancy in order to block the effect of the PTB marker on the uterus and its contents. For example, the blocked RNA can be one or more of the CFP RNA PTB biomarker described herein, where blocking the RNA can interrupt one or more myometrial preterm birth initiator genes. Also, the CFP RNA PTB biomarker being blocked can be one or more PTB biomarker miRNA, where blocking the miRNA blocks a preterm birth initiator gene.

In one embodiment, the CFP RNA PTB biomarker isolated from the pregnant mother can be normalized against a normalization sequence. If a CFP mRNA PTB biomarker, the isolated RNA can be normalized against the peptidylprolyl isomerase normalization sequence (SEQ ID NO: 1). If a CFP miRNA PTB biomarker, the isolated RNA can be normalized against one or more of normalization sequences snRNA:U6:96Aa (SEQ ID NO: 2), snRNA:U6:96Ab (SEQ ID NO: 3), and/or snRNA:U6:96Ac (SEQ ID NO: 4).

The methods described herein can also include any method of isolating RNA from blood components. This can include isolation from whole blood or blood plasma.

In one embodiment, a diagnostic kit can be provided that includes sequences to identify one or more of these CFP miRNA PTB biomarkers and/or one or more of these CFP mRNA PTB, biomarkers. These sequences can be the sequences of the Sequence Listing having SEQ ID NOs: 5-300 and/or primers and/or probes thereof. The primers and probes can be at least substantially unique for these CFP RNA PTB biomarker sequences with adequate hybridization thereto for the methods and protocols described herein. The primers and/or probes of the CFP RNA PTB biomarkers recited in the Sequence Listing can also be considered to be CFP RNA PTB biomarkers for the purpose of the invention as these primers and/or probes target to and hybridize with select specific sequences within the CFP RNA PTB biomarkers of the Sequence Listing. The RNA biomarkers can be configured to be in nucleic acid format, such as RNA, DNA, or RNA/DNA hybrid. The diagnostic kit can include individual compositions that each have a single CFP RNA PTB biomarker, or a single composition can include one or more of these CFP miRNA PTB biomarkers and/or one or more of these CFP mRNA PTB biomarkers. The CFP RNA PTB biomarkers can be provided with or without a label, such as a visual label or radiolabel. The CFP RNA PTB biomarker can be provided on a chip or a card configured for use in nucleic acid detection and/or quantification and/or qualification, which chip or card can be configured as a microarray. One or more sample spots on a microarray can one or more of these CFP miRNA PTB biomarkers and/or one or more of these CFP mRNA PTB biomarkers and/or the primers and/or probes thereof. For example, the microarray can have one spot with one of the primer and/or probe CFP miRNA PTB biomarkers and/or one of the primer and/or probe CFP mRNA PTB biomarkers. Such a microarray can have one or more CFP RNA PTB biomarker spots, which spots can be reaction wells or the like. For example, the microarray can be configured as an Affymetrix microarray card or any advancement in technology reasonably related thereto. The incorporation of these CFP RNA PTB biomarkers in the various microarray products allows them to be more readily used for plasma-derived samples, and in repeated measures of CFP RNA PTB biomarkers.

In one embodiment, a CFP RNA PTB biomarker can be a nucleic acid that contains or consists of the sequence which defines the CFP RNA PTB biomarker target or complement thereof. The CFP RNA PTB biomarker can be identical to one of SEQ ID NOs: 5-18 and/or 5-106 and/or 19-106 and/or 5-300 and/or 107-300 and/or 107-142 and/or 143-300, or can be a complement thereof, sense or antisense, as well as a sequence that hybridizes therewith under suitable conditions as well as primers and/or probes therefore. For the purposes of this invention, the primers and/or probes of the recited sequences can be considered to be CFP RNA PTB biomarkers as they are used to target the particular RNA produced within a subject. The primers and probes will be complementary to the sequences of SEQ ID NOs: 5-300, as these sequences are the targets. The CFP RNA PTB biomarker can have perfect complementarity or greater than or about 95% complementarity, greater than or about 90% complementarity, greater than or about 85% complementarity, or greater than or about 80% complementarity with the sequences recited or the probes and/or primers thereof. The CFP RNA PTB biomarker can be continuous or it can have one or more bulges or mismatches upon hybridization. The CFP RNA PTB biomarker can also include one or more chemical modifications, such as a 2' carbon modification. The CFP RNA PTB biomarker may or may not form an overhang upon hybridization. The CFP RNA PTB biomarker can include a sequence from about 15 nucleotides to the full sequence, from about 16 nucleotides to about 100 nucleotides, from about 17 nucleotides to about 50 nucleotides, from about 18 nucleotides to about 30 nucleotides, from about 19 nucleotides to about 25 nucleotides, or from about 20 to about 22 nucleotides in sequence of or complement to one of SEQ ID NOs: 5-18 and/or 5-106 and/or 19-106 and/or 5-300 and/or 107-300 and/or 107-142 and/or 143-300. The CFP RNA PTB biomarker can include a unique sequence segment of the full sequence having a length as described.

In one embodiment, the methods described herein can be performed with exon and miRNA microarrays, and can quantitate their levels using high throughput PCR. The RNA can be obtained from one or more pregnant women at least by 12 weeks of pregnancy until delivery. The RNA biomarkers can be validated using a high throughput, customized PCR card having the PTB biomarkers as described herein. The PTB biomarkers from one group of women can be validated against a second group of randomly selected women with PTB and or control women that do not have PTB or that have a term birth.

In one embodiment, the CFP mRNA/miRNA PTB biomarkers can be manipulated in presence or amount in order to modify myometrial Ca²⁺ flux that is mediated by myometrial PTB genes. It has now been found that CFP RNA PTB biomarkers that were significantly increased or decreased in women destined for PTB may be manipulated to modify myometrial Ca²⁺ flux which in turn regulates myometrial contractility. For example, the expression of the CFP mRNA APOA-4 ((SEQ ID NO: 66) *Homo sapiens* apolipoprotein A-IV (APOA4), mRNA, accession number NM_000482) increased myometrial intracellular Ca²⁺ flux. Other CFP RNA PTB biomarkers may be similarly used for manipulation of myometrial function.

### RNA PURIFICATION

Existing RNA isolation techniques can yield enough CFP RNA for an array, but not for the needed PCR validation of the hundreds of genes identified by the array unless the plasma volume is high. This explains the common practice of using solid tissues (e.g. placenta, myometrium, cervix) to identify candidates and then hope to individually quantitate them in plasma using Q-rtPCR.

Accordingly, the present invention provides a method that in one process separates intact RNA, including mRNA and miRNA, DNA and protein. The process is based on a phenol/guanidium isothiocyanate/glycerol phase separation and results in large quantities of high quality CFP nucleic acid with total RNA yields of 1.5-30 ug or more from only 2 mL of plasma. This amount is more than enough for array technology and the performance of numerous PCR reactions using a clinically practical, single patient sample.

The RNA isolation method described herein allows for the isolation of 1.5 micrograms to 7 micrograms of CFP RNA from a 2 mL sample, which is more than enough for both array use and PCR validation. The method can include obtaining: 2 mL or more of sample from a subject, such as plasma; DEPC-treated Water (Ambion); Ethanol (Sigma); Chloroform (Sigma); 3M, pH: 5.5 Sodium Acetate (Ambion); Phonel (Sigma); Guanidium isothiocyanate (Sigma); Glycerol (Sigma); Aliquot 2 mL of sample (e.g., plasma) from one patient sample into 8 tubes, 250 uL plasma in each tube. The RNA purification is conducted as follows: spin plasma at 200x g for 5 minutes at 4 C; add 750 uL phenol/guanidium isothiocyanate/glycerol lysis buffer per 2 mL sample, and vortex samples vigorously for 15 seconds and incubate them for 5 min; add 200 uL chloroform per sample and vortex sample vigorously and incubate at room temperature for 10 min; centrifuge the samples at 10,000x g for 15 minutes at 4 C, and obtain upper aqueous phase for RNA isolation, and lower red/phenol/chloroform phase can be used for DNA and Protein isolation; transfer 300 uL upper aqueous phase carefully without disturbing the interphase into a fresh tube, and add 1/10 volume of 3M Sodium acetate (pH: 5.5) (30uI) plus 3 volumes of 100% iced cold ethanol at 900 uL to each tube (note: 2 mL plasma from one patient sample can result in 13-14 tubes); incubate the tubes overnight at -20°C; centrifuge at 12,000x g for 75 minute (e.g., 4°C ), and remove all liquid; add 50 uL ice cold 80% ethanol to each tube, and then wash the pellet, then transfer all of the sample tubes into one tube, add then add 300-350 mL ice cold ethanol to make the ethanol an amount of about 1 mL; centrifuge at 12,000x g for 60 minutes at 4°C; remove all liquid, and set at 37°C to dry for 40 minutes; re-suspend the pellet in about 20-40 uL DEPC water, and incubate at 56°C for 10 minutes to dissolve RNA, and then put the RNA sample in ice for 30 minutes; and using 2 uL RNA, take OD at 260 nm and 280 nm to determine sample concentration and purity.

### MODULATING PTB GENES

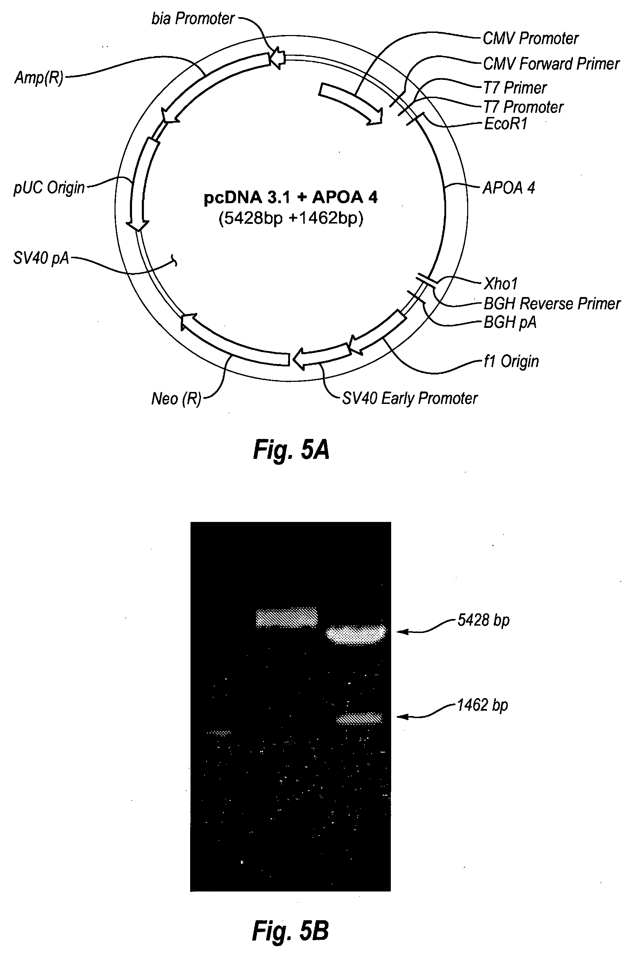

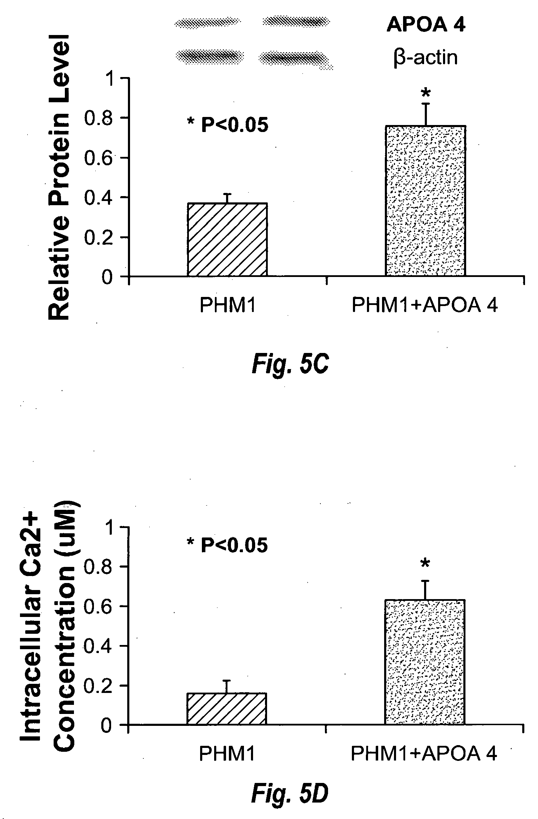

In one embodiment, the present invention can modulate CFP RNA in order to regulate intracellular Ca2⁺ flux via their effect on myometrial preterm initiator genes. For example, the CFP RNA can be used to regulate myometrial contractility. It was determined that there was an interaction between 4 CFP mRNA PTB biomarkers (e.g., PSME2, NAMPT, APOA1 and APOA 4) and 6 myometrial PTB initiator genes (e.g., IFNG, CD3E, HLA-DOA, NDRG4, VPS33A and ABCA7), and between 1 PTB marker CFP miRNA (miRLET7 G) and 1 PTB Initiator gene (SORCS). This finding supports the possibility CFP mRNA and/or miRNA PTB biomarkers could be used to alter the transcription and/or translation of myometrial preterm initiator genes. It was found that 7 PTB initiator genes that were identified to be associated with these markers could all directly or indirectly be linked to Ca²⁺ flux. A pcDNA 3.1 vector was constructed with the full length of the APOA4 mRNA (Figure 5A). Successful gene vector cloning was confirmed by EcoR1 and Xho1 restriction enzyme digests (Figure 5B). The vector was transferred into immortalized pregnant human myometrial cells PHM1, and the APOA4 was overexpressed in the PHM1 cells (Figure 5C). Over expression of the APOA4 protein dramatically increased intracellular Ca²⁺ as shown in Figure 5D. Since APOA4 is not normally expressed in cultured myometrial cells, it is conceived that the APOA4 effect on intracellular Ca²⁺ is mediated by local myometrium initiator genes. Accordingly, it is conceived that Ca²⁺ flux can be modulated with the other CFP mRNA PTB biomarkers (e.g., PSME2, NAMPT, APOA1), and the CFP miRNA PTB biomarker (e.g., miRLET7 G). This approach could be used to identify drugs that target and modulate the CFP RNA PTB biomarkers described herein or later developed. This approach can therefore be used in methods of treating women in need of labor induction, but resistant to existing methods. Also, a similar approach can be used in methods of treating women in need of labor inhibition or prevention, but resistant to existing methods.

### METHODS

Maternal Plasma: isolated in EDTA as previously described; aliquoted and stored at -80° C.

Plasma RNA Isolation: Plasma RNA is isolated by a proprietary method developed this past year. The plasma sample is lysed by phenol/guanidium isothiocyanate/glycerol buffer, and RNA, DNA and protein isolated from different aqueous phases, then precipitated using a series of proprietary chemical solutions, the pellets cleaned and resuspended in 20-40 uL DEPC water, incubated at 56 °C for 10 min to dissolve RNA, and stored at -80°C RNA yield, purity and integrity are identified by Nanospectrometer and Aligent Bio-analyzer.

Affymetrix Microarray: Affymetrix whole genome transcript and miRNA microarrays are run. Microarray QC evaluation can be performed. Each protocol is as instructed by the manufacturer.

High-throughput miRNA/mRNA Gene Validation: The ABI VIIA™ 7 is used for high-throughput mRNA/miRNA gene quantification using an ABI customized array card system. The system allows for 384 Real-time PCR reactions in 2 hours using one PCR array card (picture insert). In general, the TagMan microRNA/RNA reverse transcription kit is used to create the cDNA pool. A megaplex RT primers pool is generated based on each validated gene, and cDNA synthesized under the following thermal cycling conditions: 16°C for 2 minutes, 42°C for 1 minute, 50°C for 1 second for 40 cycles, then hold 85°C for 5 minutes. A preamplification reaction is used to enlarge gene signals. PreAmp primer pools are prepared for each validated gene. TaqMan PreAmp Master Mix will be used. PreAmplification reactions are performed under the following conditions: 95°C or 10 minutes, 55°C for 2 minutes, 72°C for 2 minutes, then 12 cycle of 95°C for 15 seconds and 60°C for 4 minutes, then 99.9°C for final denaturing step. PreAmplified cDNAs are used as the template. Validation parameters are designed and selected based on customized gene sequences.

Myometrial Cell Culture: Primary myometrial cell culture and immortalized pregnant myometrial cell are cultured using standard procedures. All primary cell lines can be derived from a large fundal myometrial biopsy from a single patient at term prior to labor.

Construction of recombinant plasmid pcDNA-CFP genes: Vector construction is used for CFP marker gene over expression. The target genes are selected from the array and IPA analyses (Preliminary Results). The purified CFP gene products and plasmid eukaryotic expression vector (pcDNA 3.1) are digested with EcoR1 and Xho1. The ligation reaction is conducted according to the manufacturer's protocol (Invitrogen). The final plasmid pcDNA-CFP genes are then transformed into E.coli JM 2163. The ligation products are cultured with LB medium containing ampicillin (100 ug/ml) overnight. Afterward, the recombinant plasmid is extracted from colony transformants prior to being identified by digesting with EcoR1 and Xho1, and confirmed by agarose gel electrophoresis. Lipofectamine will be used for transfection. Over expression of CFP marker genes will be proven by Western blot and Real-time PCR. The technique is established in our laboratory (Figure 6A).

Ca²⁺ Flux Measurement: myometrial cells are suspended in 2 mL of DMEM media consisting of 10% fetal bovine serum, 30 µg fungizone, 1% penicillin-streptomycin, 0.5% L-glutamine in DMEM (all ingredients from GIBCO Life Technologies), warmed to 37°C, and then plated on 25-mm glass coverslips. Cells are incubated in fura 2-AM (2 x 10⁶ mol/L) for 40 minutes at room temperature in the dark. Coverslips are inserted into an open microincubator (PDMI-2, Medical Systems) and attached to the stage of an inverted microscope (Nikon EclipseTE2000, Nikon;Melville, NY). The microincubator is maintained at 37°C with a bipolar temperature controller (TC-202, Medical Systems). Images are collected using Nikon EZ-C1 software and processed with Volocity imaging software (Improvision Inc., Lexington, MA). Data are expressed as a ratio of emitted fluorescence at 510 nm in cells excited at 340 and 380 nm. Responses to genes or their respective vehicles (DMSO, ethanol) are analyzed by directly transfected siRNA or gene expression vector. Changes in the 340/380 nm emission ratios are recorded. Ca²⁺ influx rate is calculated based on previous reports. Data are expressed as a ratio of emitted fluorescence at 510 nm in cells excited at 340 and 380 nm. Responses to genes or their respective vehicles (DMSO, ethanol) are analyzed by directly transfected siRNA or gene expression vector. Changes in the 340/380 nm emission ratios are recorded and Ca2⁺ influx rate is calculated.

One skilled in the art will appreciate that, for this and other processes and methods disclosed herein, the functions performed in the processes and methods may be implemented in differing order. Furthermore, the outlined steps and operations are only provided as examples, and some of the steps and operations may be optional, combined into fewer steps and operations, or expanded into additional steps and operations without detracting from the essence of the disclosed embodiments.

The present disclosure is not to be limited in terms of the particular embodiments described in this application, which are intended as illustrations of various aspects. Many modifications and variations can be made without departing from its spirit and scope, as will be apparent to those skilled in the art. Functionally equivalent methods and apparatuses within the scope of the disclosure, in addition to those enumerated herein, will be apparent to those skilled in the art from the foregoing descriptions. Such modifications and variations are intended to fall within the scope of the appended claims. The present disclosure is to be limited only by the terms of the appended claims, along with the full scope of equivalents to which such claims are entitled. It is to be understood that this disclosure is not limited to particular methods, reagents, compounds compositions or biological systems, which can, of course, vary. It is also to be understood that the terminology used herein is for the purpose of describing particular embodiments only, and is not intended to be limiting.

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity.

It will be understood by those within the art that, in general, terms used herein, and especially in the appended claims (e.g., bodies of the appended claims) are generally intended as "open" terms (e.g., the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an" (e.g., "a" and/or "an" should be interpreted to mean "at least one" or "one or more"); the same holds true for the use of definite articles used to introduce claim recitations. In addition, even if a specific number of an introduced claim recitation is explicitly recited, those skilled in the art will recognize that such recitation should be interpreted to mean at least the recited number (e.g., the bare recitation of "two recitations," without other modifiers, means at least two recitations, or two or more recitations). Furthermore, in those instances where a convention analogous to "at least one of A, B, and C, etc." is used, in general such a construction is intended in the sense one having skill in the art would understand the convention (e.g., " a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (e.g., " a system having at least one of A, B, or C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

In addition, where features or aspects of the disclosure are described in terms of Markush groups, those skilled in the art will recognize that the disclosure is also thereby described in terms of any individual member or subgroup of members of the Markush group.

As will be understood by one skilled in the art, for any and all purposes, such as in terms of providing a written description, all ranges disclosed herein also encompass any and all possible subranges and combinations of subranges thereof. Any listed range can be easily recognized as sufficiently describing and enabling the same range being broken down into at least equal halves, thirds, quarters, fifths, tenths, etc. As a non-limiting example, each range discussed herein, can be readily broken down into a lower third, middle third and upper third, etc. As will also be understood by one skilled in the art all language such as "up to," "at least," and the like include the number recited and refer to ranges which can be subsequently broken down into subranges as discussed above. Finally, as will be understood by one skilled in the art, a range includes each individual member. Thus, for example, a group having 1-3 cells refers to groups having 1, 2, or 3 cells. Similarly, a group having 1-5 cells refers to groups having 1, 2, 3, 4, or 5 cells, and so forth.

A unique segment of a sequence in a sequence listing is a specific sequence segment that is found within the recited sequence of the SEQ ID NO, and substantially absent in the rest of the RNA transciptome. That is, the unique segment of the sequence in the Sequence Listing identified by the SEQ ID NO can be used as a probe or a primer that is specific for that SEQ ID NO. The techniques available for identifying a primer or a probe available to one of ordinary skill in the art can be used to identify one or more unique segments of each SEQ ID NO recited in the Sequence Listing.

## Claims

1. Use of a composition comprising a nucleic acid having a cell free plasma (CFP) RNA preterm birth (PTB) biomarker sequence including or complementary to one or more of SEQ ID NOs: 5-300 or a probe or primer specific thereto, the nucleic acid being present in an amount sufficient for use in a nucleic acid diagnostic protocol in the diagnosis of susceptibility to PTB.

2. The use according to claim 1, wherein the composition further comprises a nucleic acid having a normalization sequence including or complementary to one or more of SEQ ID NOs: 1-4 and 301-303 or a probe or primer specific thereto, the nucleic acid being present in an amount sufficient for use in a nucleic acid normalization protocol.

3. The use according to claim 2, wherein the composition has two or more of the normalization sequences.

4. The use according to any of claims 1 to 3, wherein the composition comprises one or more of:
a nucleic acid having a CFP RNA PTB biomarker sequence including or complementary to one or more of SEQ ID NOs: 5-18 or a probe or primer specific thereto
a nucleic acid having a CFP RNA PTB biomarker sequence including or complementary to one or more of SEQ ID NOs: 19-106 or a probe or primer specific thereto;
a nucleic acid having a CFP RNA PTB biomarker sequence including or complementary to one or more of SEQ ID NOs: 107-142 or a probe or primer specific thereto; or
a nucleic acid having a CFP RNA PTB biomarker sequence including or complementary to one or more of SEQ ID NOs: 143-300 or a probe or primer specific thereto.

5. The use according to any of claims 1 to 4, wherein the composition has two or more of the CFP RNA PTB biomarker sequences.

6. A method for predicting susceptibility of a pregnant woman to preterm birth (PTB), comprising:
obtaining cell free plasma (CFP) RNA transcriptome nucleic acids from a sample of cell free plasma from the pregnant woman; and
determining a change in the CFP RNA transcriptome in the sample obtained from the pregnant woman, wherein the change is indicative for susceptibility to PTB,
wherein the CFP RNA transcriptome includes a CFP RNA PTB biomarker having one or more sequences from one or more of SEQ ID NOs: 5-300 or complement thereof; and wherein the change in the CFP RNA transcriptome comprises at least one of:
an up-regulation of a CFP RNA PTB biomarker having one or more sequences from one or more of SEQ ID NOs: 5-7, 19-41 and 107-142 or complement thereof; or
a down-regulation of a CFP RNA PTB biomarker having one or more sequences from one or more of SEQ ID NOs: 8-18, 42-106 and 143-300 or complement thereof.

7. The method according to claim 6, wherein the sample includes CFP RNA at about 16 weeks to about 32 weeks of pregnancy.

8. The method according to claim 6 or 7, wherein the CFP RNA transcriptome includes a CFP RNA PTB biomarker having one or more sequences from one or more of SEQ ID NOs: 19-106 or complement thereof.

9. The method according to any of claims 6 to 8, wherein the CFP RNA transcriptome includes a CFP miRNA PTB biomarker that is up-regulated in order to indicate susceptibility of PTB, the CFP miRNA PTB biomarker having a sequence of one or more of SEQ ID NOs: 5-7 or complement thereof.

10. The method according to any of claims 6 to 9, wherein the CFP RNA transcriptome includes a CFP miRNA PTB biomarker that is down-regulated in order to indicate susceptibility of PTB, the CFP miRNA PTB biomarker having a sequence of one or more of SEQ ID NOs: 8-18 or complement thereof.

11. The method according to any of claims 6 to 10, wherein the CFP RNA transcriptome includes a CFP small RNA PTB biomarker that is up-regulated in order to indicate susceptibility of PTB, the CFP small RNA PTB biomarker having a sequence of one or more of SEQ ID NOs: 19-41 and/or 107-142 or complement thereof.

12. The method according to any of claims 6 to 11, wherein the CFP RNA transcriptome includes a CFP small RNA PTB biomarker that is down-regulated in order to indicate susceptibility of PTB, the CFP small RNA PTB biomarker having a sequence of one or more of SEQ ID NOs: 42-106 and/or 143-300 or complement thereof.

13. The method according to any of claims 6 to 12, comprising one or more of:
using a primer and/or a probe that is complementary to and hybridizes to one of SEQ ID NOs: 5-106 in order to identify the change in a CFP RNA transcriptome;
using a primer and/or a probe that is includes the sequence of one of SEQ ID NOs: 107-300 in order to identify the change in a CFP RNA transcriptome;
using a primer and/or a probe that includes the sequence of one of SEQ ID NOs: 107-142 in order to identify an up-regulation of in a CFP RNA PTB biomarker; or
using a primer and/or a probe that includes the sequence of one of SEQ ID NOs: 142-300 in order to identify a down-regulation of in a CFP RNA PTB biomarker.

14. The method according to any of claims 6 to 13, comprising:
comparing the amount of one or more CFP RNA PTB biomarkers from a first sample and a second sample obtained from the pregnant woman, wherein a change in amount indicates susceptibility to PTB, wherein the first sample includes one or more CFP RNA PTB biomarkers of the CFP RNA transcriptome prior to 12 weeks of pregnancy and the second sample includes one or more CFP RNA PTB biomarkers after 12 weeks of pregnancy; and
determining at least a fold change in the amount of one or more CFP RNA PTB biomarkers of the CFP RNA transcriptome between the first sample and the second sample.

15. The method of according to any of claims 6 to 14, comprising normalizing the amount of CFP RNA PTB biomarker from the CFP RNA transcriptome by using one or more of the normalization sequences including or complementary to one or more of SEQ ID NOs: 1-4 and 301-303 or unique segment thereof.

## Patentansprüche

1. Verwendung einer Zusammensetzung, umfassend eine Nukleinsäure mit einer Biomarker-Sequenz für Frühgeburten (PTB) aus zellfreier Plasma(CFP)-RNA, die eine oder mehrere aus SEQ ID NR: 5-300 oder eine(n) dafür spezifische(n) Sonde oder Primer umfasst oder komplementär dazu ist, wobei die Nukleinsäure in einer Menge vorhanden ist, die zur Verwendung in einem Nukleinsäure-Diagnoseprotokoll zur Diagnose der Anfälligkeit für eine Frühgeburt ausreicht.

2. Verwendung nach Anspruch 1, wobei die Zusammensetzung ferner eine Nukleinsäure umfasst, die eine Normalisierungssequenz aufweist, die eine oder mehrere aus SEQ ID NR: 1-4 und 301-303 oder eine(n) dafür spezifische(n) Sonde oder Primer umfasst oder komplementär dazu ist, wobei die Nukleinsäure in einer Menge vorhanden ist, die zur Verwendung in einem Nukleinsäure-Normalisierungsprotokoll ausreicht.

3. Verwendung nach Anspruch 2, wobei die Zusammensetzung zwei oder mehr der Normalisierungssequenzen aufweist.

4. Verwendung nach einem der Ansprüche 1 bis 3, wobei die Zusammensetzung eine oder mehrere der Folgenden umfasst:
eine Nukleinsäure mit einer CFP-RNA-Frühgeburts-Biomarkersequenz, die eine oder mehrere aus SEQ ID NR: 5-18 oder eine(n) dafür spezifische(n) Sonde oder Primer umfasst oder komplementär dazu ist;
eine Nukleinsäure mit einer CFP-RNA-Frühgeburts-Biomarkersequenz, die eine oder mehrere aus SEQ ID NR: 19-106 oder eine(n) dafür spezifische(n) Sonde oder Primer umfasst oder komplementär dazu ist;
eine Nukleinsäure mit einer CFP-RNA-Frühgeburts-Biomarkersequenz, die eine oder mehrere aus SEQ ID NR: 107-142 oder eine(n) dafür spezifische(n) Sonde oder Primer umfasst oder komplementär dazu ist; oder
eine Nukleinsäure mit einer CFP-RNA-Frühgeburts-Biomarkersequenz, die eine oder mehrere aus SEQ ID NR: 143-300 oder eine(n) dafür spezifische(n) Sonde oder Primer umfasst oder komplementär dazu ist.

5. Verwendung nach einem der Ansprüche 1 bis 4, wobei die Zusammensetzung zwei oder mehr der CFP-RNA-Frühgeburts-Biomarkersequenzen aufweist.

6. Verfahren zum Vorhersagen der Anfälligkeit einer schwangeren Frau für eine Frühgeburt (PTB), umfassend:
Gewinnen von Nukleinsäuren des zellfreien Plasma(CFP)-RNA-Transkriptoms aus einer Probe aus zellfreiem Plasma der schwangeren Frau; und
Bestimmen einer Veränderung des CFP-RNA-Transkriptoms in der Probe, die von der schwangeren Frau gewonnen wurde, wobei die Veränderung die Anfälligkeit für eine Frühgeburt anzeigt, wobei das CFP-RNA-Transkriptom einen CFP-RNA-Frühgeburts-Biomarker umfasst, der eine oder mehrere Sequenzen aus einer oder mehreren von SEQ ID NR: 5-300 oder ein Komplement davon aufweist; und
wobei die Veränderung des CFP-RNA-Transkriptoms wenigstens eines von Folgendem umfasst:
eine Hochregulierung eines CFP-RNA-Frühgeburts-Biomarkers, der eine oder mehrere Sequenzen aus einer oder mehreren von SEQ ID NR: 5-7, 19-41 und 107-142 oder ein Komplement davon aufweist; oder
eine Runterregulierung eines CFP-RNA-Frühgeburts-Biomarkers, der eine oder mehrere Sequenzen aus einer oder mehreren von SEQ ID NR: 8-18, 42-106 und 143-300 oder ein Komplement davon aufweist.

7. Verfahren nach Anspruch 6, wobei die Probe CFP-RNA von etwa der 16. Schwangerschaftswoche bis etwa der 32. Schwangerschaftswoche umfasst.

8. Verfahren nach Anspruch 6 oder 7, wobei das CFP-RNA-Transkriptom einen CFP-RNA-Frühgeburts-Biomarker umfasst, der eine oder mehrere Sequenzen aus einer oder mehreren von SEQ ID NR: 19-106 oder ein Komplement davon aufweist.

9. Verfahren nach einem der Ansprüche 6 bis 8, wobei das CFP-RNA-Transkriptom einen CFP-miRNA-Frühgeburts-Biomarker umfasst, der hochreguliert ist, um die Anfälligkeit für eine Frühgeburt anzuzeigen, wobei der CFP-miRNA-Frühgeburts-Biomarker eine Sequenz aus einer oder mehreren von SEQ ID NR: 5-7 oder ein Komplement davon aufweist.

10. Verfahren nach einem der Ansprüche 6 bis 9, wobei das CFP-RNA-Transkriptom einen CFP-miRNA-Frühgeburts-Biomarker umfasst, der runterreguliert ist, um die Anfälligkeit für eine Frühgeburt anzuzeigen, wobei der CFP-miRNA-Frühgeburts-Biomarker eine Sequenz aus einer oder mehreren von SEQ ID NR: 8-18 oder ein Komplement davon aufweist.

11. Verfahren nach einem der Ansprüche 6 bis 10, wobei das CFP-RNA-Transkriptom einen Frühgeburts-Biomarker aus kleiner CFP-RNA umfasst, der hochreguliert ist, um die Anfälligkeit für eine Frühgeburt anzuzeigen, wobei der Frühgeburts-Biomarker aus kleiner CFP-RNA eine Sequenz aus einer oder mehreren von SEQ ID NR: 19-41 und/oder 107-142 oder ein Komplement davon aufweist.

12. Verfahren nach einem der Ansprüche 6 bis 11, wobei das CFP-RNA-Transkriptom einen Frühgeburts-Biomarker aus kleiner CFP-RNA umfasst, der runterreguliert ist, um die Anfälligkeit für eine Frühgeburt anzuzeigen, wobei der Frühgeburts-Biomarker aus kleiner CFP-RNA eine Sequenz aus einer oder mehreren von SEQ ID NR: 42-106 und/oder 143-300 oder ein Komplement davon aufweist.

13. Verfahren nach einem der Ansprüche 6 bis 12, umfassend eines oder mehrere von Folgendem:
Verwenden eines Primers und/oder einer Sonde, der oder die komplementär zu einer von SEQ ID NR: 5-106 ist und daran hybridisiert, um die Veränderung eines CFP-RNA-Transkriptoms zu identifizieren;
Verwenden eines Primers und/oder einer Sonde, der oder die die Sequenz von einer von SEQ ID NR: 107-300 umfasst, um die Veränderung eines CFP-RNA-Transkriptoms zu identifizieren;
Verwenden eines Primers und/oder einer Sonde, der oder die die Sequenz von einer von SEQ ID NR: 107-142 umfasst, um eine Hochregulierung eines CFP-RNA-Frühgeburts-Biomarkers zu identifizieren; oder
Verwenden eines Primers und/oder einer Sonde, der oder die die Sequenz von einer von SEQ ID NR: 142-300 umfasst, um eine Runterregulierung eines CFP-RNA-Frühgeburts-Biomarkers zu identifizieren.

14. Verfahren nach einem der Ansprüche 6 bis 13, umfassend:
Vergleichen der Menge von einem oder mehreren CFP-RNA-Frühgeburts-Biomarkern aus einer ersten Probe und einer zweiten Probe, die von der schwangeren Frau gewonnen wurden, wobei eine Veränderung der Menge eine Anfälligkeit für eine Frühgeburt anzeigt, wobei die erste Probe einen oder mehrere CFP-RNA-Frühgeburts-Biomarker des CFP-RNA-Transkriptoms vor der 12. Schwangerschaftswoche umfasst und die zweite Probe einen oder mehrere CFP-RNA-Frühgeburts-Biomarker nach der 12. Schwangerschaftswoche umfasst; und
Bestimmen von wenigstens einer relativen Veränderung der Menge von einem oder mehreren CFP-RNA-Frühgeburts-Biomarkern des CFP-RNA-Transkriptoms zwischen der ersten Probe und der zweiten Probe.

15. Verfahren nach einem der Ansprüche 6 bis 14, umfassend das Normalisieren der Menge des CFP-RNA-Frühgeburts-Biomarkers aus dem CFP-RNA-Transkriptom durch Verwendung von einer oder mehreren der Normalisierungssequenzen, die eine oder mehrere von SEQ ID NR: 1-4 und 301-303 oder ein eindeutiges Segment davon umfassen oder komplementär dazu sind.

## Revendications

1. Utilisation d'une composition comprenant un acide nucléique qui a une séquence biomarqueur de naissance prématurée (PTB) à ARN de plasma acellulaire (CFP), comprenant une ou plusieurs des SEQ ID n° : 5 à 300, ou bien une sonde ou amorce spécifique de celle(s)-ci, ou bien étant complémentaire à celle(s)-ci, l'acide nucléique étant présent en une quantité suffisante pour être utilisée dans un protocole diagnostique à acide nucléique dans le diagnostic de la susceptibilité à une PTB.

2. Utilisation selon la revendication 1, dans laquelle la composition comprend en outre un acide nucléique ayant une séquence de normalisation, qui comprend une ou plusieurs des SEQ ID n° : 1 à 4 et 301 à 303, ou bien une sonde ou amorce spécifique de celle(s)-ci, ou bien étant complémentaire à celle(s)-ci, l'acide nucléique étant présent en une quantité suffisante pour être utilisée dans un protocole de normalisation d'un acide nucléique.

3. Utilisation selon la revendication 2, dans laquelle la composition a deux ou plusieurs des séquences de normalisation.

4. Utilisation selon l'une quelconque des revendications 1 à 3, dans laquelle la composition comprend un ou plusieurs des éléments suivants :
un acide nucléique ayant une séquence biomarqueur de PTB à ARN de CFP, qui comprend une ou plusieurs des SEQ ID n° : 5 à 18, ou bien une sonde ou amorce spécifique de celle(s)-ci, ou bien étant complémentaire à celle(s)-ci;
un acide nucléique ayant une séquence biomarqueur de PTB à ARN de CFP, qui comprend une ou plusieurs des SEQ ID n° : 19 à 106, ou bien une sonde ou amorce spécifique de celle(s)-ci, ou bien étant complémentaire à celle(s)-ci;
un acide nucléique ayant une séquence biomarqueur de PTB à ARN de CFP, qui comprend une ou plusieurs des SEQ ID n° : 107 à 142, ou bien une sonde ou amorce spécifique de celle(s)-ci , ou bien étant complémentaire à celle(s)-ci;
un acide nucléique ayant une séquence biomarqueur de PTB à ARN de CFP, qui comprend une ou plusieurs des SEQ ID n° : 143 à 300, ou bien une sonde ou amorce spécifique de celle(s)-ci, ou bien étant complémentaire à celle(s)-ci.

5. Utilisation selon l'une quelconque des revendications 1 à 4, dans laquelle la composition a deux ou plusieurs des séquences biomarqueurs de PTB à ARN de CFP.

6. Procédé de prédiction de la susceptibilité d'une femme enceinte à accoucher prématurément (PTB), comprenant:
obtenir des acides nucléiques d'un transcriptome d'ARN de plasma acellulaire (CFP) à partir d'un échantillon de plasma acellulaire provenant de la femme enceinte ; et
déterminer un changement dans le transcriptome d'ARN de CFP dans l'échantillon obtenu à partir de la femme enceinte, dans lequel le changement est une indication d'une susceptibilité à une PTB,
dans lequel le transcriptome d'ARN de CFP comprend un biomarqueur de PTB à ARN de CFP ayant une ou plusieurs séquence(s) provenant d'une ou de plusieurs des SEQ ID n° : 5 à 300 ou un complément de celle(s)-ci ; et
dans lequel le changement dans le transcriptome d'ARN de CFP comprend au moins un élément parmi :
une régulation à la hausse d'un biomarqueur de PTB à ARN de CFP, ayant une ou plusieurs séquence(s) provenant d'une ou de plusieurs des SEQ ID n° : 5 à 7, 19 à 41 et 107 à 142 ou un complément de celle (s)-ci ; ou
une régulation en baisse d'un biomarqueur de PTB à ARN de CFP, ayant une ou plusieurs séquence (s) provenant d'une ou de plusieurs des SEQ ID n° : 8 à 18, 42 à 106 et 143 à 300 ou un complément de celle(s)-ci.

7. Procédé selon la revendication 6, dans laquelle l'échantillon comprend de l'ARN d'un CFP à environ 16 semaines jusqu'à environ 32 semaines de grossesse.

8. Procédé selon la revendication 6 ou la 7, dans laquelle le transcriptome d'ARN de CFP comprend un biomarqueur de PTB à ARN de CFP ayant une ou plusieurs séquence(s) provenant d'une ou de plusieurs des SEQ ID n° : 19 à 106 ou un complément de celle(s)-ci.

9. Procédé selon l'une quelconque des revendications 6 à 8, dans laquelle le transcriptome d'ARN de CFP comprend un biomarqueur de PTB à miARN de CFP qui est régulé à la hausse dans le but d'indiquer une susceptibilité à une PTB, le biomarqueur de PTB à miARN de CFP ayant une séquence provenant d'une ou de plusieurs des SEQ ID n° : 5 à 7 ou un complément de celle(s)-ci.

10. Procédé selon l'une quelconque des revendications 6 à 9, dans laquelle le transcriptome d'ARN de CFP comprend un biomarqueur de PTB à miARN de CFP qui est régulé en baisse dans le but d'indiquer une susceptibilité à une PTB, le biomarqueur de PTB à miARN de CFP ayant une séquence provenant d'une ou de plusieurs des SEQ ID n° : 8 à 18 ou un complément de celle(s)-ci.

11. Procédé selon l'une quelconque des revendications 6 à 10, dans laquelle le transcriptome d'ARN de CFP comprend un biomarqueur de PTB à petit ARN de CFP qui est régulé à la hausse dans le but d'indiquer une susceptibilité à une PTB, le biomarqueur de PTB à petit ARN de CFP ayant une séquence provenant d'une ou de plusieurs des SEQ ID n° : 19 à 41 et/ou 107 à 142 ou un complément de celle(s)-ci.

12. Procédé selon l'une quelconque des revendications 6 à 11, dans laquelle le transcriptome d'ARN de CFP comprend un biomarqueur de PTB à petit ARN de CFP qui est régulé en baisse dans le but d'indiquer une susceptibilité à une PTB, le biomarqueur de PTB à petit ARN de CFP ayant une séquence provenant d'une ou de plusieurs des SEQ ID n° : 42 à 106 et/ou 143 à 300 ou un complément de celle(s)-ci.

13. Procédé, selon l'une quelconque des revendications 6 à 12, comprenant une ou plusieurs des étapes consistant à :
utiliser une amorce et/ou une sonde qui est complémentaire à l'une des SEQ ID n° : 5 à 106 et qui s'hybride avec celle-ci, dans le but d'identifier le changement dans un transcriptome d'ARN de CFP ;
utiliser une amorce et/ou une sonde qui inclut la séquence de l'une des SEQ ID n° : 107 à 300, dans le but d'identifier le changement dans un transcriptome d'ARN de CFP ;
utiliser une amorce et/ou une sonde qui inclut la séquence de l'une des SEQ ID n° : 107 à 142, dans le but d'identifier une régulation à la hausse d'un biomarqueur de PTB à ARN de CFP ; ou
utiliser une amorce et/ou une sonde qui inclut la séquence de l'une des SEQ ID n° : 142 à 300, dans le but d'identifier une régulation en baisse d'un biomarqueur de PTB à ARN de CFP.

14. Procédé, selon l'une quelconque des revendications 6 à 13, comprenant:
comparer la quantité d'un ou de plusieurs biomarqueur(s) de PTB à ARN de CFP à partir d'un premier échantillon et d'un deuxième échantillon obtenus de la femme enceinte, dans lequel un changement dans la quantité indique une susceptibilité à une PTB, dans lequel le premier échantillon comprend un ou plusieurs biomarqueur(s) de PTB à ARN de CFP du transcriptome d'ARN de CFP avant 12 semaines de grossesse et le deuxième échantillon comprend un ou plusieurs biomarqueur (s) de PTB à ARN de CFP après 12 semaines de grossesse ; et
déterminer au moins un changement d'ordre de grandeur dans la quantité d'un ou de plusieurs biomarqueur(s) de PTB à ARN de CFP du transcriptome d'ARN de CFP entre le premier échantillon et le deuxième échantillon.

15. Procédé, selon l'une quelconque des revendications 6 à 14, comprenant la normalisation de la quantité de biomarqueur de PTB à ARN de CFP à partir du transcriptome d'ARN de CFP en utilisant une ou plusieurs des séquences de normalisation, qui comprennent une ou plusieurs des SEQ ID n° : 1 à 4 et 301 à 303 ou étant complémentaires à celle(s)-ci, ou bien un segment unique de celle(s)-ci.
